# EUROPEAN PATENT SPECIFICATION

(11) **EP 1 469 905 B1**
(45) Date of publication and mention of the grant of the patent: **11.03.2015**
(21) Application number: 03702116.9
(22) Date of filing: 14.01.2003
(51) Int. Cl.: B29C 65/16

(54) **LASER WELDABLE MEDICAL TUBING ASSEMBLY**
LASERSCHWEISSBARE MEDIZINISCHE ANORDNUNG VON RÖHREN
ASSEMBLAGE DE TUBULURES MEDICALES SOUDABLES PAR LASER

(30) Priority: 31.01.2002 US 61835; 20.09.2002 US 251683; 20.09.2002 US 251682
(43) Date of publication of application: 27.10.2004
(60) Divisional application: 10075417.5
(73) Proprietor: Baxter International Inc., Deerfield, IL 60015 (US)
(72) Inventor: ALBERTI, James, Odessa, FL 33556 (US); BIEWER, John, Palm Harbor, FL 34683 (US); DIN, Shahid, Palm Harbor, FL 34685 (US); DO, David, Valrico, FL 33594 (US); GILBERT, Rick, Palm Harbor, FL 34685 (US); PENNINGTON, David, Fox Lake, IL 60020 (US); ROMACK, Joe, Clearwater, FL 33761 (US); LANDHERR, Frank, Cary, IL 60013 (US); SHANG, Sherwin, Vernon Hills, IL 60061 (US); YANG, Tahua, Woodridge, IL 60517 (US)
(74) Representative: Potter Clarkson LLP
(86) International application number: PCT/US2003/001107
(87) International publication number: WO 2003/063940

## Description

### BACKGROUND OF THE INVENTION

The present invention relates to laser-weldable flexible tubing that is particularly well suited for use in the delivery of therapeutic solutions such as peritoneal dialysis solutions.

It is known to use medical containers with tubing for various medical procedures such as kidney dialysis, intravenous delivery of therapeutic fluids, delivery of nutritional fluids, delivery of blood, blood components, and blood substitutes. Fluid containers and tubing are also widely used in other industries such as the food industry and the chemical industries.

For example, flexible medical tubings are used in systems for treating renal disease. In renal failure of any cause, there are several physiological derangements. The balance of water, minerals and the excretion of daily metabolic load is no longer possible in renal failure. During renal failure, toxic end products of nitrogen metabolism (urea, creatinine, uric acid and others) can accumulate in blood and tissues.

Kidney failure and reduced kidney function have been treated with dialysis. Dialysis removes waste, toxins and excess water from the body that would otherwise have been removed by normal functioning kidneys. Dialysis treatment for replacement of kidney functions is critical to many people because the treatment is life saving. One who has failed kidneys could not continue to live without replacing at least the filtration functions of the kidneys. Hemodialysis and peritoneal dialysis are two types of dialysis therapies commonly used to treat loss of kidney function.

In general, hemodialysis treatment removes waste, toxins, and excess water from the patient's blood. The patient is connected to a hemodialysis machine and the patient's blood is pumped through the machine. Catheters are inserted into the patient's veins and arteries to connect the blood flow to and from the hemodialysis machine. As blood passes through a dialyzer in the hemodialysis machine, waste, toxins, and excess water are removed from the patient's blood and the blood is infused back into the patient. Many tubes are used in the process that must be connected or disconnected. Hemodialysis treatment lasts several hours and is generally performed in a treatment center about three or four times per week.

Peritoneal dialysis, typically, utilizes a dialysis solution, or dialysate, which is infused into a patient's peritoneal cavity. The dialysate contacts the patient's peritoneal membrane in the peritoneal cavity. Waste, toxins and excess water pass from the patient's bloodstream through the peritoneal membrane and into the dialysate. The transfer of waste, toxins and water from the bloodstream into the dialysate occurs due to diffusion and osmosis, i.e., there is an osmotic gradient across the membrane. The spent dialysate is drained from the patient's peritoneal cavity to remove the waste, toxins and water from the patient. After the spent dialysis is drained, it is replaced with a fresh dialysate solution.

While the present invention has application in connecting or disconnecting tubes for medical procedures, the following discussion focuses, as an example, on a particular tube connection and disconnection processes performed during peritoneal dialysis. Numerous tubes are used in the process that must be connected or disconnected. In peritoneal dialysis, patients have a catheter implanted in their peritoneal cavity with an end protruding from the patient. The protruding end of the catheter terminates with a section of tubing known as a transfer set. The transfer set is typically made from a silicone material and must be periodically replaced say every several months. The transfer set is provided to connect the patient to dialysate fluid bags or discharge bags. The transfer set typically has a spike that connects to an access port positioned in a tube associated with the drain bag or dialysate solution bag (dialysate set). In general, the patient manually stabs the port with the spike to connect the transfer set to the dialysate set. The patient connects the tube in the transfer set to a drain to allow spent dialysate fluid to drain from the peritoneal cavity. Next, the patient is connected to a bag of fresh dialysate and manually infuses the fresh dialysate through the catheter and into the patient's peritoneal cavity. When the patient completes treatment, the port is pulled off the spike and a cap is placed on the spike until the patient is ready for the next treatment. When the patient disconnects the catheter from the fresh dialysate bag, the dialysate dwells within the peritoneal cavity to draw waste, toxins and excess water from the patient's bloodstream to the dialysate solution. After the dwell period, the patient repeats the manual dialysis procedure and drains the spent dialysate from the peritoneal cavity.

Accordingly, during dialysis treatments such as those described above, the patient is required to connect and disconnect the catheter and transfer set from the fill or drain line (or tube) a number of times. Some devices are available today to assist the patient during the process when using specialized sterilization equipment. However, by and large, these connections and disconnections are performed manually.

One such device, incorporates a heated wafer or hot knife that physically contacts the tubing to cut it by melting the tube and joining two tubes together or melt-sealing the tube ends. Typically, heated wafer applications involve a "melt and wipe" process. In peritoneal dialysis, for example, a patient must drain spent dialysate or replenish his/her peritoneal cavity with fresh dialysate. To this end, the patient must connect the transfer set tubing to a tube extending from either a drain bag or a bag containing fresh dialysate. In one "melt and wipe" process, the transfer set tubing is bent in a U or V-shape to fit into a U or V-shaped tube holder. Similarly, the bag-side tube is bent in a U or V-shape to fit into another U or V-shaped tube holder adjacent the first tube holder. A heated wafer moves across the space between the two tube holders and physically contacts the tubing at the bend junction of the U-shape or V-shape. As the heated wafer contacts the tubing, it melts the tube at the bend junction of the U-shape or V-shape. The wafer then wipes the melted tubing material and removes the material from the area between the tube holders. The two holders are brought together and two connections are made. In the first connection, the transfer set tubing is connected to the bag-side tube and the dialysis process is ready to begin. In the second connection, the wasted tube material from the transfer set tubing and the bag-side tube is connected together and discarded.

In order to disconnect the patient from the bag, hot knives are used to cut the tube. An example of a known disconnecting process with the hot knife involves two tubes that are placed side by side across two tube holders. One of the tubes is a short tube having two sealed ends. Generally, the tube holders include a ridge at one end of the tube holder to flatten a portion of the tube to stop fluid flow. The hot knife severs each tube into two pieces. After the hot knife cuts the tube, one of the tube holders moves in relation to the other tube holder. The tubing is "swapped," realigned with one of the cut portions of the short tube, and connected to it - thus, a disconnection is made between the patient and the bag.

These devices have a relatively low level of reliability due to the inconsistency in melting and cutting processes. This inconsistency can result in imperfect seals, leading to leaks, bacterial infiltration and, ultimately, the patient may well experience, among other things, infection or peritonitis. Also, none of these known methods inspect the integrity of the weld formed between the two tube ends during the connection process. Thus, users must rely on their own visual inspection of the weld.

Moreover, these devices are not user friendly. Often times, patients that need dialysis treatment are visually or otherwise impaired. For example, some dialysis patients experience manual dexterity problems. Many of the known processes involve a great deal of human interaction with loading the tubes into the tube holders. Also, the equipment should be cleaned and the heated wafer replaced after each use to avoid contamination. Thus, this is a difficult process for visually impaired patients and those with poor manual dexterity.

WO 01/68362 describes a multilayer film (10). The film has a first layer of a blend of a first component selected from the group: (1) ethylene and α-olefin copolymers having a density of less than about 0.915 g/cc, (2) ethylene copolymerized with lower alkyl acrylates, (3) ethylene copolymerized with lower alkyl substituted alkyl arcylates and (4) ionomers, the first component being present in an amount from about 99% to about 55% by weight of the blend, a second component in an amount by weight of the blend from about 45% to about 1 % and consists of one or more polymers of the group: (1) propylene containing polymers, (2) polybutene polymers, (3) polymethylpentene polymers, (4) cyclic olefin containing polymers and (5) bridged polycyclic hydrocarbon containing polymers and a second layer (4) attached to the first layer (12).

WO 00/20157 describes a method of forming a weld between workpieces over a joint region. The method comprises: exposing the joint region to incident radiation having a wavelength outside the visible range so as to cause melting of the surface of one or both workpieces at the joint region, and allowing the melted material to cool thereby welding the workpieces together. A radiation absorbing material is provided at the joint region in one of the workpieces or between the workpieces which has an absorption band matched to the wavelength of the incident radiation so as to absorb the incident and generate heat from the melting process.

WO 82/04016 describes a connector for joining sealingly incompatible materials comprising telescopically related first and second cylindrical layers, said first layer and said second layer comprised of polymers selected to melt or deform at different temperatures, said first layer being capable of sealing to a first material, and said second layer being capable of sealing to a second material sealingly incompatible with said first material. The first layer may be essentially composed of polyvinyl chloride and the second layer may be essentially comprised of poly(ethyl-vinyl acetate).

### SUMMARY OF THE INVENTION

The present invention provides improved medical devices. The present invention provides a tubing assembly according to claim 1, a medical fluids delivery assembly according to claim 4 and claim 14. Methods of performing medical procedures are also described. In general, the method and device include a connecting process and a disconnecting process, each of which use laser technology. In the connecting process, the laser forms a weld between two flexible tubes. In the disconnecting process, the laser seals a tube to form two sealed end tube segments from a single tube.

A method for providing a connection between two tube ends of flexible material is described. The method includes the steps of directing a laser beam at the two tube ends and heating each tube end; forcing the heated tube ends together; forming a weld having weld characteristics; and comparing the weld characteristics to a weld profile. The method may further include the step of determining whether the two tube ends are acceptable for connection. The step of determining whether the ends are acceptable for connection includes confirming that one tube end is the patient-side and the other tube end is the bag-side tube. An optics assembly may be used to direct the laser used to heat each tube end.

A method of connecting two tube ends of flexible material includes the steps of providing two tube holders, each tube holder receiving one of the two tube ends; directing a laser beam at the two tube ends to sterilize the two tube ends; urging the two tube holders together so that the two tube ends contact each other; and forming a weld between the two tube ends. The method may further include the step of comparing the weld characteristics to a weld profile and determining whether the weld characteristics are at least ecual to the weld profile.

A device for making a connection between two tube ends includes a laser beam. The device further includes a laser optics assembly capable of changing the a direction of the laser beam so that the laser beam strikes the tube ends. A pair of tube holders are further provided, each adapted to receive a flexible tube end and adapted to urge the two tube ends together after the two tube ends are aseptically heated via the laser beam, to join the heated tube ends together to form a weld. The laser optics assembly may further include a prism movably mounted between the two tube ends, and a collimator located between the laser unit and the prism. A "Y"-shaped optical component may be added adjacent to the laser unit which is adapted to split the laser beam and direct the beam towards each tube end. A light pipe may be included in the laser optics assembly.

A device is described for connecting two thermoplastic ends together. The device includes two tube holders, each tube holder having an aperture adapted to receive one of the two tube ends. The device further provides a laser unit spaced in relation to the tube holders which is capable of projecting a laser beam towards the two tube ends in order to sterilize each end and connect the two tube ends together. A sensor is provided near the tube holders to analyze the connection between the two tube ends: A tracking system may be connected to the two tube holders and capable of moving the two tube holders together to form a weld between the two tube ends. The tracking system includes an edge detector for sensing the position of each of the two tube ends in the two tube holders. A heat sensor may be provided for monitoring the temperature near at least one of the two tube ends during the connection process.

A device is described for forming a sterile connection between two flexible tube ends. The device includes a housing having a back and two slots, each slot adapted to receive one of the flexible tube ends. A pair of guide wires are provided positioned within the housing near each slot, each of the guides directs one of the flexible tube ends into the housing. The device also includes a laser unit positioned within the housing, and a pair of tube holders positioned within the housing, each of the pair of tube holders adapted to receive the tube end from one of the pair of guides. The tube holders are capable of manipulating the flexible tube ends so that each tube end faces the laser unit for heating. The tube holders bring the heated tube ends together to form a weld and subsequently release the resulting welded tube. The device may also include a sensor in communication with the guides for triggering the guides to an "on" state when the tube ends are present in each of the slots.

Methods for providing a connection between two thermoplastic tubes, each tube having a sealed end are also described. The method includes the steps of providing a housing adapted to receive the two thermoplastic tubes; providing a laser unit within the housing; loading the sealed end of each thermoplastic tube into the housing; manipulating the thermoplastic tubes within the housing so that each sealed end faces the laser unit; sterilizing and opening the sealed ends by energizing the laser unit; manipulating the thermoplastic tubes again so that the now opened ends are aligned with each other; and welding the two tube ends together via the laser unit.

A method for disconnecting a flexible tube is also described. The method includes the steps of compressing the flexible tube at an area along the tube; striking a laser beam at the compressed area; sealing the compressed area; and separating the flexible tube into two tubes, each tube having a sealed end.

A method for providing an aseptic disconnection of a flexible tube is described. The method includes the steps of providing a housing that has an interior section adapted to receive the flexible tube; providing a laser unit in the interior section of the housing; selecting an area along the flexible tube; crimping the area of the flexible tube; sealing the area via the laser unit; and separating the tube into two tube segments at the area, each of the tube segments having a sealed end.

A device is described for disconnecting a flexible tube. The device includes a laser unit having an on and off state; a pair of guides, each guide adapted to receive the flexible tube and move the flexible tube; a crimping device in between the pair of guides, the crimping device initially compresses the flexible tube while the laser unit is in the off state, the crimping device further pinches and seals the flexible tube when the laser is in the on state; and the guides move in opposite direction from one another resulting in two sealed segments of flexible tube.

A device is described for disconnecting a flexible tube. The device includes a housing having a lid. A hammer and an anvil aligned with the hammer in the housing. The hammer and the anvil compress the flexible tube. A laser unit mounted in the housing, the laser unit is energized after the flexible tube is compressed and de-energized after a seal forms in the compressed tube. A separator that creates a tension at the seal and splits the flexible tube into two tube segments, each tube segment having a sealed end.

A device is described to connect two flexible tube ends together and to disconnect a single flexible tube. The device includes a laser unit having an on and off state, the laser unit emitting a laser beam in the on state; a laser optics assembly capable of changing a direction of the laser beam; a pair of tube holders, each tube holder adapted to receive a flexible tube end, the tube ends being aseptically heated via the laser beam, and the tube holders subsequently join the heated tube ends together to form a weld; a pair of guides, each guide adapted to receive the single flexible tube and move the flexible tube; a crimping device in between the pair of guides, the crimping device initially compresses a flexible tube while the laser unit is in the off state, the crimping device further pinches and seals the flexible tube when the laser is in the on state; and the guides move in opposite direction from one another resulting in two sealed segments of flexible tube.

A method of providing dialysis treatment to a patient is described. The method includes the step of sealing a first tube end and a second tube end of medical tubing together via a laser unit.

A polymer blend is described for fabricating a laser-weldable article having a first component of a material not thermally responsive to a laser beam and selected from the group consisting of polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers; a second component of a laser responsive material having low solubility in an aqueous medium and present in an amount by weight of from about 20 ppm to about 2000 ppm; and the blend being sufficiently thermally responsive to exposure to a laser beam having a wavelength within a range of wavelengths from about 700 nm to about 1500 nm to at melt upon exposure to the laser beam for a short period of time:

The present invention provides tubing assembly comprising: (a) a tubing having a fluid outlet and a sidewall comprising a layer from a polymer blend comprising a first component which is a material not thermally responsive to a laser beam and selected from the group consisting of polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers; a second component of a laser responsive material having low solubility in an aqueous medium; and the blend melts upon to exposure to a laser beam having a wavelength within a range of wavelengths from 700 nm to 1500 nm for a short period of time; and (b) an end cap film covering the fluid outlet which is hermetically sealed to the tubing and is a polymeric material having a monolayer structure or a multilayer structure, wherein the polymeric material is a blend comprising a component selected from the group consisting of: (1) ethylene and α-olefin copolymers having a density of less than 0.915 g/cc, (2) ethylene and lower alkyl acrylate copolymers, (3) ethylene and lower alkyl substituted alkyl acrylate copolymers and (4) ionic polymers; and a component selected from the group consisting of : (1) propylene containing polymers, (2) butene containing polymers, (3) polymethyl pentene containing polymers, (4) cyclic olefin containing polymers and (5) bridged polycyclic hydrocarbon containing polymers.

The second component of the polymer blend of the sidewall of the tubing may be present in an amount by weight of from 20 ppm to 500 ppm.

The film is a polymeric material having a monolayer structure or a multilayer structure comprising a first component selected from from the group consisting of: (1) ethylene and α-olefin copolymers having a density of less than 0.915 g/cc, (2) ethylene and lower alkyl acrylate copolymers, (3) ethylene and lower alkyl substituted alkyl acrylate copolymers and (4) ionic polymers. and a second component selected frcm the group consisting of: (1) propylene containing polymers, (2) butene containing polymers, (3) polymethyl pentene containing polymers, (4) cyclic olefin containing polymers and (5) bridging polycyclic hydrocarbon containing polymers.

The present invention also provides an end cap film comprising a layer comprising a first blend of a polymeric component and a laser responsive component, wherein tho polymeric component is a second blend of from 99% to 50% by weight of the first component and from 50% to 1% of the second components; and wherein the film melts upon exposure to a laser beam having a wavelength from 700nm to 1500 nm for a short period of time,

The present invention further provides a film wherein th polymeric component is a second blend comprising by weight of from 35% to 45% of a first ethylene and ∀-olefin copolymer having a density of less than 0.900 g/cc, from 20% to 30% of a second ethylene and α-olefin copolymer having a density of higher than 0.900 g/cc but less than 0.910 g/cc, and from 30% to 40% of a polypropylene.

Also described herein is a laser weldable multiple lumen tubing having a first lumen and a second lumen each having a layer from a polymer blend. The layer has a first component of a material not thermally responsive to a laser beam and selected from the group consisting of polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers, a second component in an amount by weight of from about 20 ppm to about 2,000 ppm of a laser responsive material having low solubility in aqueous medium and the layer being sufficiently thermally responsive to exposure to a laser beam having a wavelength within a range of wavelengths from about 700 nm to about 1500 nm to melt a portion of the sidewall upon exposure to the laser beam for a short period of time.

A polymer blend for fabricating a laser-weldable article is described. The polymer blend has a first component of a material not thermally responsive to a laser beam and selected from the group consisting of polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers; a second component of a laser responsive material having low solubility in an aqueous medium and present in an amount by weight of from about 20 ppm to about 2000 ppm; and the blend being sufficiently thermally responsive to exposure to a laser beam having a wavelength within a range of wavelengths from about 700 nm to about 1500 nm to melt upon exposure to the laser beam for a short period of time.

### BRIEF DESCRIPTION OF THE FIGURES:

FIG. 1 is a perspective view of a housing FIGS. 2A through 2D are perspective views of a connection and disconnection device as described herein.
FIGS. 3A and 3B are perspective views of another tube holder as described herein.
FIGS. 4A through 4H are schematic plan views of the device embodiment in FIGS. 2A through 2D.
FIGS. 5A through 5C are schematic cross-sectional views of an embodiment of a sealed end tube of the present invention.
FIG. 6 is a schematic plan view of a protective film according to principles of the present invention.
FIG. 7 is a schematic plan view of a device as described herein.
FIG. 8 is a perspective view of an optical assembly.
FIGS. 9A and 9B are schematic plan views of an optical assembly with a laser unit.
FIGS. 10A and 10B are perspective views of a laser assembly without an optical assembly.
FIGS. 11a and 11b are respectively cross-sectional views of a monolayer, non-PVC, laser weldable tubing and a multiple layer tubing having the monolayer tubing as a layer therein and a multilumen tubing.
FIG. 12 is a cross-sectional view of a capped tubing assembly.
FIG. 13 is a cross-sectional view of a coupler.
FIG. 14 is a cross-sectional view of a tubing, and coupler assembly.
FIG. 15 is a plan view of a medical fluids container connected to a non-PVC laser-weldable tubing.
FIG. 16 is a cross-sectional view of a laser-weldable tubing connected through a coupler device to a tubing from a transfer set
FIG. 17 is a cross-sectional view of a dual lumen tubing.
FIG. 18 is a cross-sectional view of a dual lumen tubing with the individual lumen concentrically disposed with respect to one another.
FIG. 19 is a cross-sectional view of a multiple lumen tubing.

### DETAILED DESCRIPTION OF THE INVENTION:

Although the present invention can be made in many different forms, the presently preferred embodiments are described in this disclosure and shown in the attached drawings. This disclosure exemplifies the principles of the present invention and does not limit the broad aspects of the invention only to the illustrated embodiments.

Generally, the present invention relates to the aseptic connection and disconnection of tubing. Such tubing can be advantageously used to transfer fluid or blood to and from the human body. A device that opens sealed tube ends and connects the opened tube ends together is described. Moreover, the device disconnects a tube and reseals the tubing. All of these processes use laser generated heat and provide a connection or disconnection that is aseptic or sterile.

### The Device

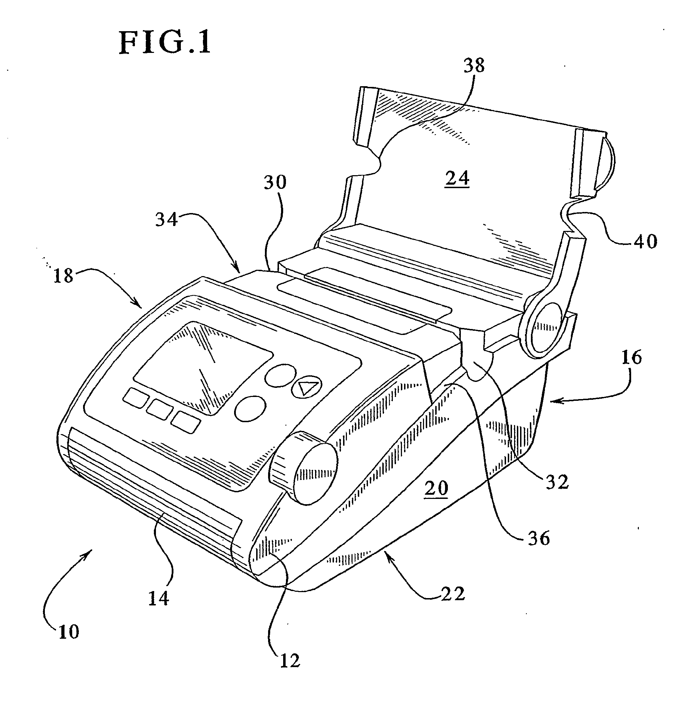

FIG. 1 shows a device **10** including a housing **12** that has a front **14,** a back **16,** and two sides **18, 20** there between. The housing **12** also includes a bottom **22** and a lid or door **24.** The four sides **14, 16, 18, 20** and bottom **22** define an interior area A. Two slots or openings **30,32** are located at upper ends **34, 36** of two sides **18, 20** of the housing **12.** The door **24** is hinged to the back **16** of the housing **12.** The door **24** may be hinged in any number of ways to allow it to be easily opened and shut. The door includes a locking mechanism (not shown) to lock the door closed when the device is in operation. In an embodiment, the door **24** also has two slots **38, 40** that align with the slots **30, 32 to** create an area (not shown) for loading and unloading tubing **50** which will be described in further detail below.

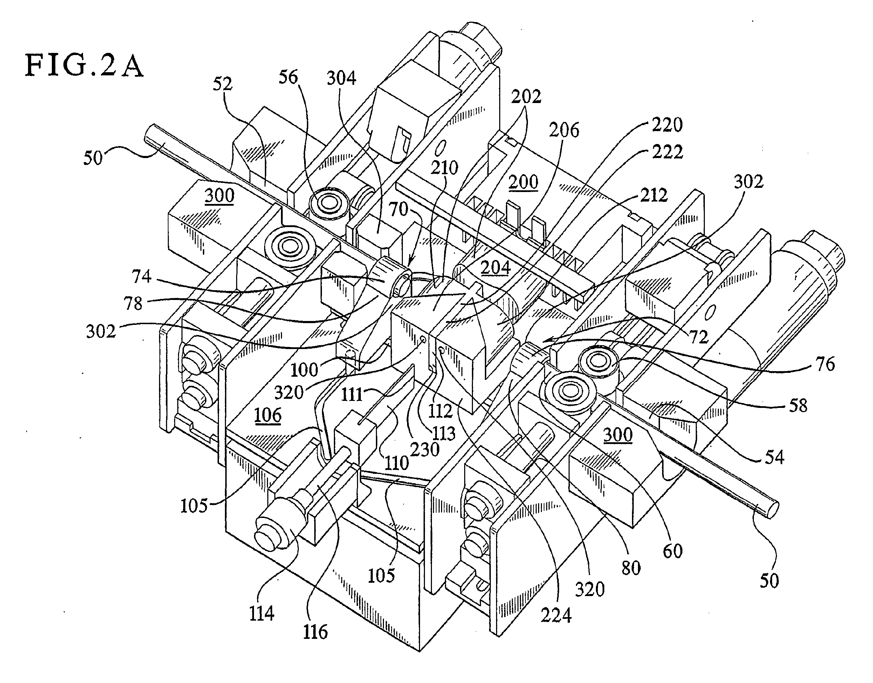

FIGS. 2A through 2D show the inside of the device **10.** Inside the housing **12** are two passageways **52**, **54.** In an example, the passageways **52, 54** are funneled. Each passageway leads to a guide **56, 58.** The guides **56, 58** receive the tubing **50** and advance the tubing **50** within the housing **12.** The guides are, preferably, pinch rollers, however, various types of guides or threading devices, may be used. The guides **56, 58** preferably crimp the tubing **50** as it is fed into the device **10.** (See FIG. 2A, Ref. No. **58**). This crimping purges fluid from a portion **60** of the tube **50** that progresses past the guides **56, 58** into the device.

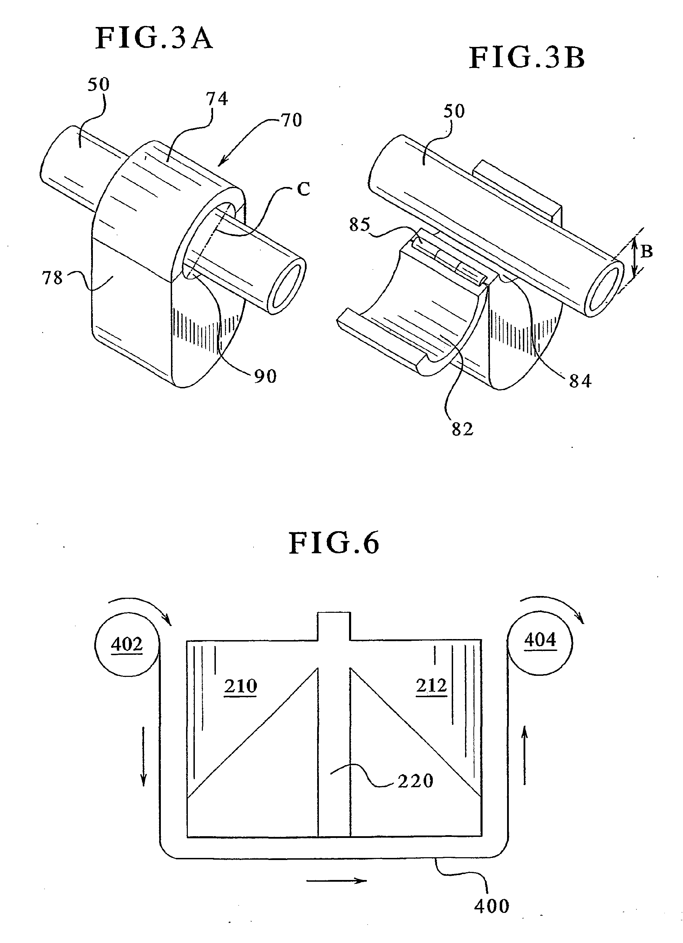

FIGS. 2A through 2D also show a pair of tube holders **70, 72** aligned with guides **56, 58** in the housing **12.** FIGS. 3A and 3B show.an enlarged view of another tube holder **70.** As shown in FIGS. 2A through 2D and 3A and 3B, each tube holder **70, 72** has a first part **74, 76** and a second part **78, 80,** respectively. Each first and second part **74, 76** and **78, 80** has a recess or groove **82, 84** that corresponds with an outer diameter B of the tubing **50.** The first part **74, 78** is movably attached to the second part **76, 80** via a hinge **85** or similar mechanism. When the tube holders **70, 72** are in the closed position, an aperture **90** is formed extending through the holder **70**, **72.** A diameter C of this aperture **90** is slightly smaller than the outer diameter B of the tube **50.** In this way, the tubing **50** is fed through the guides **56, 58** and received in the tube holders **70, 72.** An inside surface of the tube holders **70, 72** may be tapered (not shown). The aperture **90** may be 'slightly tapered toward the center of the device **10.** In this example, the diameter C of the aperture **90** facing the inside of the device is smaller than a diameter of the aperture facing the guides **70, 72.** When in the closed position, the tube holders **70, 72** close with sufficient force to grip, but not flatten, the tubing. In addition, if necessary, the aperture **90** uniformly compresses the tubing **50** and forces the tubing to maintain a cylindrical shape. This may be necessary if, for example, the tubing **50** is not cylindrical due to storage conditions of the tubing or prior sterilization methods, e.g., steam sterilization or ETO sterilization, which may cause the tubing **50** to coil and not be perfectly round.

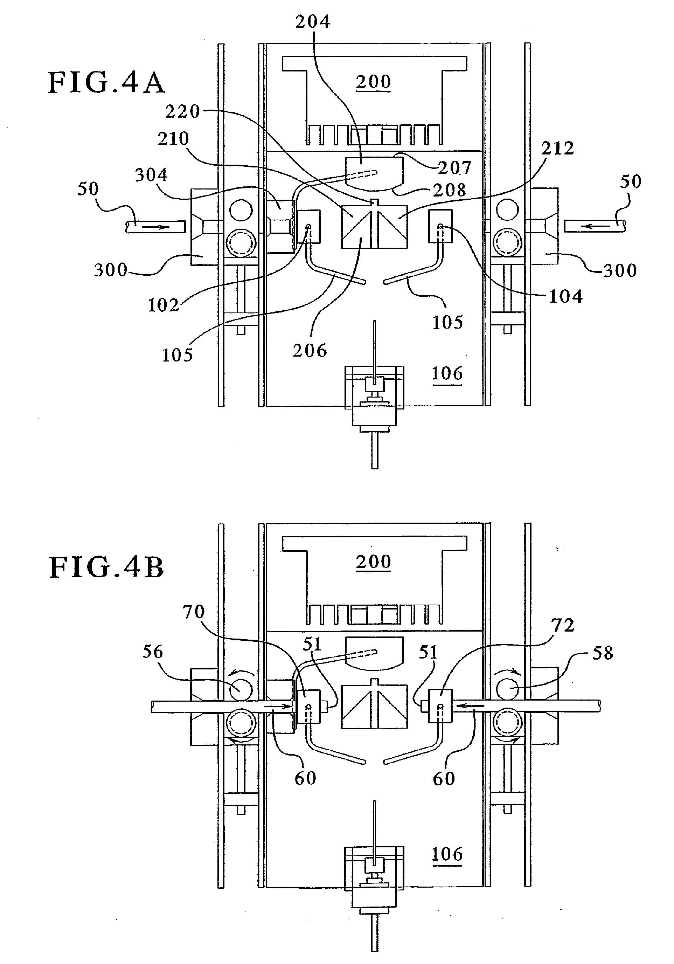

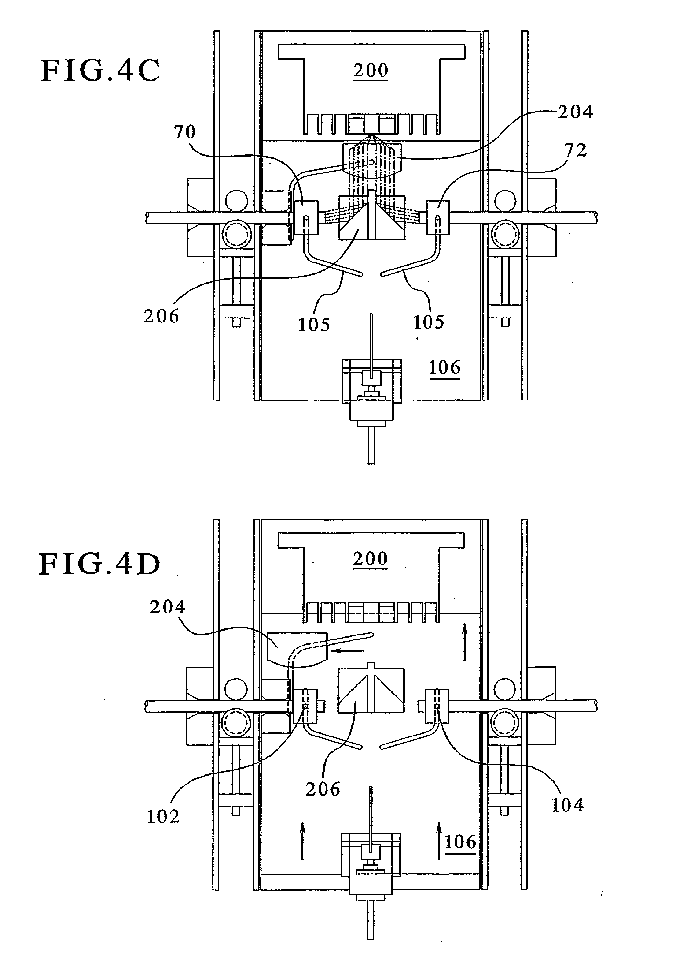

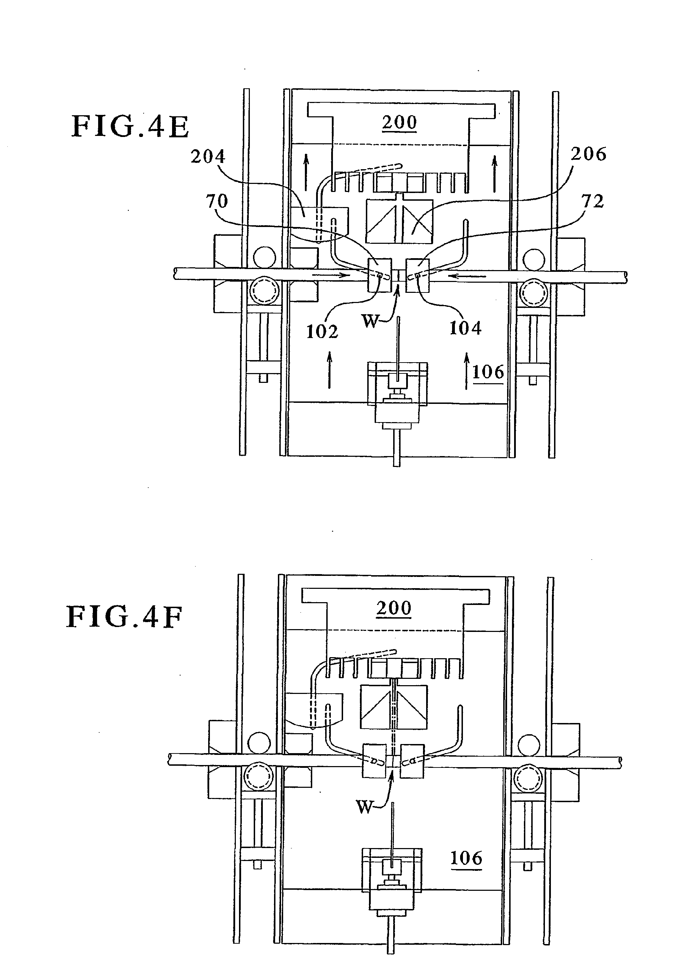

The tube holders **70, 72** may be mounted on a bar **100.** Each tube holder has a guide arm **102, 104** associated with it. The guide arms **102, 104** extend below the bar **100** to a track **105** in a plate **106.** As described in more detail below in conjunction with FIGS. 4A through 4H, the plate **106** moves back and forth within the housing **12.** As the plate **106** moves to the back **16,** the tube holders **70, 72** move in a straight line toward each other to the center of the device **10** (See FIGS. 4D and 4E). The guide arms **102, 104** may be, for example, a lead screw or lever/cam/slot mechanism or a combination of any of these. With the assistance of the guide arms **102, 104,** the tube holders **70, 72** pull or push the tube **50** within the housing **12;** thus, manipulating the tubing **50** to a desired position, e.g., sterilizing and opening two sealed ends of tubing, and connecting the two ends together, or disconnecting, sterilizing and sealing the ends of a single tube.

As will be described in detail below, the device **10** also includes a hammer **110** and an anvil **112.** The hammer **110** and anvil **112** are used during the disconnecting process of the tubing **50.** The hammer **110** is movably mounted to a motor **114** via a shaft **116.** In an embodiment, the hammer **110** moves forward and backward along the shaft **116** in the housing. As the hammer moves forward, a front part **111** of the hammer contacts a surface **113** of the anvil **112.** The hammer **110** may be made from a metal, ceramic, or even rigid plastic material.

### Laser Optics

The device also includes a laser unit **200.** The laser unit **200** may be a semiconductor diode laser which can be a single laser diode or a laser array of diodes. However, other types of lasers can be used in the invention. For example, Argon, CO₂ or YAG lasers may be used. The laser characteristics, e.g., wavelength of the laser, should be evaluated to determine the corresponding characteristics of the tubing **50** to be used in the application. The laser unit **200** may have an optical assembly to direct a controlled laser beam to the desired location for the connecting or disconnecting processes.

FIGS. 2A through 2D and 4A through 4H show an optical assembly **202**. In this example, the optical assembly **202** includes a collimator 204, and a reflective prism **206.** Depending on the characteristics of the laser unit **200**, a laser beam may begin to diverge as soon as it leaves the unit 200. In this scenario, the collimator **204** limits the divergence of the laser beam. Specifically, the collimator **204** has a generally flat back surface **207** that faces the laser **200.** The collimator **204** also has slightly convex front surface **208.** As the laser energy travels through the collimator **204,** the collimator refocuses the laser beam to the prism **206.** Other applications, for examples CO₂, may have a small laser beam that can be expanded by using a beam expander. The collimator **204** is, preferably, made from an acrylic material, however, other transparent or translucent materials may be used.

The prism **206** splits the laser beam and directs the split beam to the desired location, e.g., the tube ends 51, for the connection process. In order to obtain optimal laser concentration during the connection process, the design of the prism **206** is directly related to the prism location in the device **10.** The prism **206** may be between the two tube holders **70, 72.** In this example, the prism **206** is constructed from two plano convex lenses **210, 212** juxtaposed to each other.

As is further described below, the prism **206** may also include a light pipe **220** that intersects a center **222** of the prism **206.** The light pipe **220** directs the laser beam during both the connecting and disconnecting processes. In addition, the anvil **112** is along a back **224** of the prism lens **206** and, specifically, near an end **230** of the light pipe **220.**

### Tubing

In general, the material of the tubing **50** is a flexible plastic. The tubing and assemblies thereof are discussed in greater detail below. In a preferred embodiment, the material is a thermoplastic, kraton polypropylene blend, or the like. In one preferred form of the invention, a chemical additive is added that is responsive to the laser to generate heat. One particularly suitable additive can be selected from dyes. The dye is selected to absorb energy at or near the wavelength of the laser diode to promote absorption of the energy of the laser, thereby heating the tubing. Thus, the frequency of the laser selected, e.g., semiconductor diode or YAG laser, should match the specific characteristics of the dye that is added to the tube material. In some applications, e.g., CO₂ laser applications, no dye may be required because the absorption wavelength of the tube is the same as the wavelength generated by the laser.

Moreover, a second dye may be added to color code each of the tubes. Such color coding creates a machine detectable and patient detectable distinction between the tubing that is connected to the patient and the new tubing to be connected. For example, the catheter tubing that is implanted in the patient, or the transfer set connected to the catheter, may be dyed one color and the tubing that is attached to the bag of fluid may be dyed a different color. This color distinction is especially helpful for patients that are visually impaired.

### Sensors

A number of sensors **300, 302, 304,** ... are positioned within the housing **12.** It should be understood that the location of the sensors identified in the drawings is just one example. Other acceptable locations for the sensors may be accomplished depending on the layout of the components within the device **10.** These sensors detect and confirm different stages of the process, whether it is during the connection or the disconnection processes. For example, during the connection process, a sensor **300** may be employed to identify an object at the funneled pathway **52, 54.** If the object is acceptable, e.g., the tubing **50,** the sensor **300** will activate the guides **56, 58.** If the object is not acceptable, the guides are not activated. Thus, these sensors help to keep out foreign objects, and even fingers. This sensor **300** may be, for example, an absorption sensor. An absorption sensor identifies tubing **50** that has a dye. In this way, not only will the sensor keep out foreign objects but also it will identify if improper tubing is attempting to be loaded. As mentioned above, the patient's catheter (or transfer set connected to the catheter) may be a different color than the tube connected to the fluid or blood to be administered to the patient. In this way, the absorption sensor checks to make sure the patient-side and disposable (or bag-side) sealed end tubes are loaded in the pathways **52, 54.** If the user attempts to improperly load two bag-side tubes, the sensor 300 alerts the user and the user must retry the loading procedure. Depending on the application, the sensor may be set to allow certain combinations of tubing to enter the apparatus. Thus, the sensor **300** provides a safety measure to guard against improper loading.

Another sensor that may be used in the device **10** is an edge sensor **302.** The edge sensor **302** identifies when tube ends **51** extend beyond the tube holders **70, 72** during loading of the tube ends **51** into the device. Specifically, as the tube end **51** crosses the light beam path, the signal from the photo detector **302** is fed into a comparator. The sensor **302** subsequently switches the output state at the desired threshold level, e.g., when sufficient tube length extends beyond the tube holder **70, 72.** The sensor **302** may be, for example, a precision edge sensor such as a Cartesian Ovoid LED and a die mounted aperture photo detector. However, other sensing devices capable of identifying the edge of the tube **50** may be used.

Moreover, during the disconnection process, the single tube is loaded into the device for sealing and separation. A color sensor **300** or **304** checks to make sure that not all the tubing in the device is the same color. If the entire tube **50** is the same color, the device will not be able to locate the prior weld. The sensor **304** alerts the user and the patient must reload the tube **50** and try again. This occurs because the sensor **304** also determines where to disconnect the tube **50** based on the position of an existing weld **W** in the tube. Therefore, when two tubes of different color are present at the sensors **304** the existing weld **W** is somewhere in between.

After the sensors confirm the tubing **50** is loaded properly, the same or different sensor **304** determines the location of the existing weld **W** in the tube. This may be accomplished, for example, using a digital camera - like mechanism that searches for the range in the weld. The sensor **304** may be a CMOS Image Detector. Once the existing weld W is located, the sensor identifies the position for crimping, sealing and separating the tube. The sensor then activates the guides and moves the tube a predetermined distance, on the catheter side, toward the patient. For example, the existing weld may be located at position X. The sensor locates the weld and moves the tube X + 1/8" toward the patient side for the location of the cut. In this way, the sensor ensures that the section of tube containing the existing weld W is discarded. This maintains the integrity of the remaining tube in the transfer set that leads to the catheter tube implanted in the patient. In addition, the sensor provides a safety measure since making a new weld on top of an existing weld may not be sufficiently durable.

Alternatively, the sensor **304** may detect a distinction in color between the tubing based on a color coding scheme like that described above. Accordingly, the sensor identifies a color change at the area surrounding the weld.

As described in further detail below, the device **10** also includes a number of temperature or heat sensors (**320**) to maintain consistency throughout the operation of the device. These sensors **320** may be infrared sensors, such as thermopile infrared sensors. However, other sensors such as thermal couplers or thermistors may be employed. The sensors 320 are used, for example, during the connections and disconnection processes. The sensors verify the tubing is heating properly and may be calibrated to indicate a level of heat is reached for a "good weald" or "bad weld." For example, in applications where the tubing includes a dye, the beast is absorbed by the dye and, in turn, the tubing begins to melt and flow. In this way, the sensors are non-contact temperature sensors that correspond to the infrared output of the tubing as the tubing absorbs the energy from the laser.

Thus, sensors may be employed to compensate the effectiveness of the system based on efficiency of the dye concentration of the tubing, power variation or laser optic variations.

### The Method

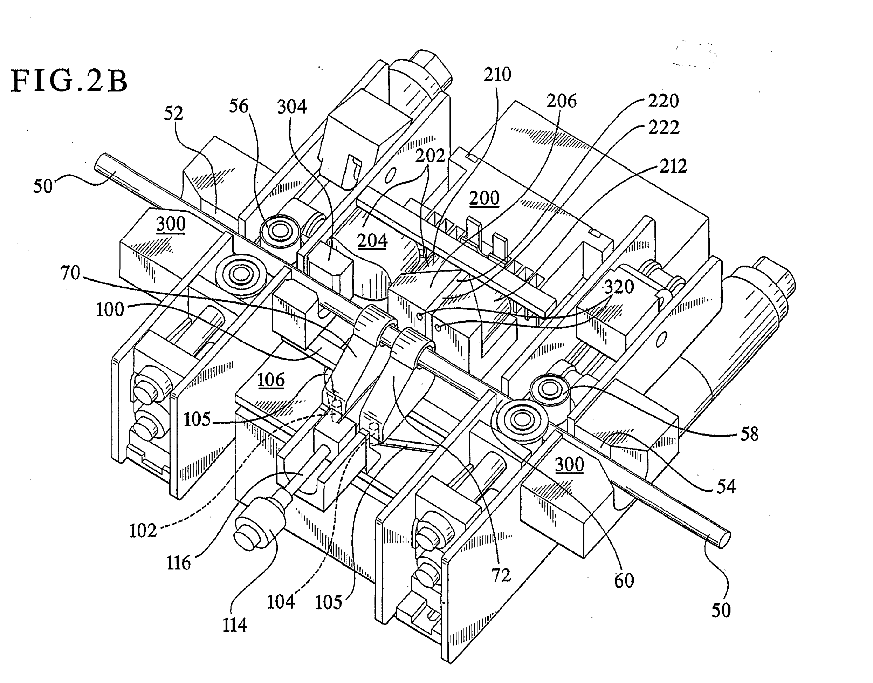

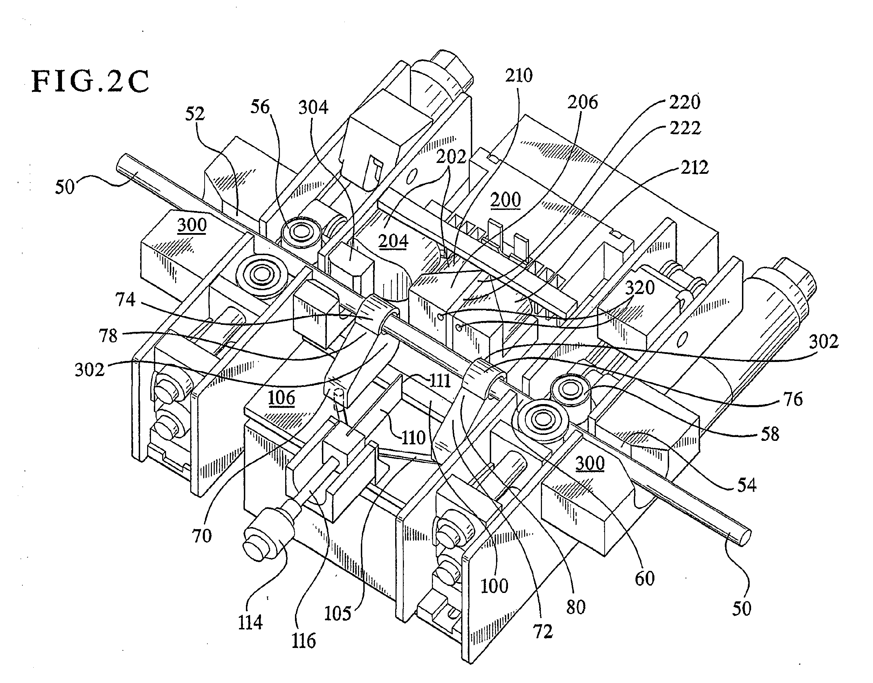

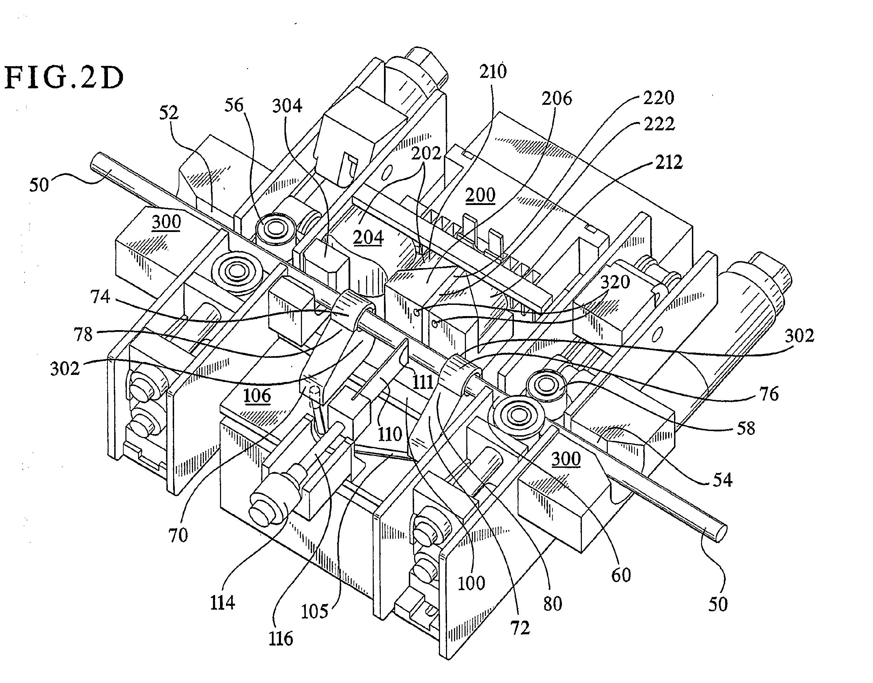

FIGS. 2A through 2D illustrate the connecting and disconnecting process as follow. Specifically, FIGS. 2A and 2B show the inventive process that connects two tube ends together. FIGS. 2C and 2D show the inventive process that disconnects the tubing. Additionally, FIGS. 4A though 4H show a simplified schematic of the connecting process.

### Method of Connecting Two Rube Ends

The method of connecting two tube ends will now be described. During the connection process, the lid **24** is closed. As shown in FIGS. 2A and 4A, the user inserts two tubes **50,** each having a sealed end **51,** into the device **10** via the loading area openings **30, 32, 38, 40.** However, it is within the scope of the invention to use at least one tube end **51** that is not sealed, but, open. In applications involving an open tube end, several types of end caps may be used to maintain the necessary sanitation levels at the inside of the tube. One type of end cap may be a sealed "drum head" that covers the end of the tube. The sealed "drum head" may be a piece of film placed over the open end of the tube and sealed around the entire face of the tube. Another example may include an open end with a vented seal over the face of the tube. A vented seal may be, for example, a perforated membrane. In this example, an end cap would be added to cover the vented end for sanitation purposes. The tubing and cap assembly will be discussed in greater detail below.

As each tube end **51** enters a respective passageway **52, 54** the sensor **300** identifies the tubing **50** and checks to make sure that one tube end **51** is the patient's tube and the other tube end **51** is the bag tube, It should be noted that it does not matter which tube is loaded into which loading area. Advantageously, the sensors **300** at the passageway **52, 54** communicate with each other to determine that one of each tubing type is loaded. When the tubing is properly loaded, the guides **56, 58** are activated. (FIG. 4B). The guides **56, 58** crimp or squish the tubing and advance each tube **end 51** into the device **10** to the tube holders **70, 72.** This crimping or squashing creates a vacuum effect in the tubing and purges fluid from the portion **60** of tubing that enters the device **10.** The precision edge sensor **302** identifies when the tube **51** extends the predetermined length beyond the holder **70, 72** and stops the guides **56, 58.**

In FIGS. 4C, the reflective prism 206 is between the tube holders **70, 72.** After each tube end **51** is loaded into its respective tube holder **70, 72,** the laser **unit 200** is activated and energy diverges from the laser source. The collimator **204** refocuses the diverting energy toward the prism lens **206.** As the energy/light strikes the reflective prism **206** it reflects into two bundles of energy. The prism lenses **210, 212** re-direct each bundle of energy at approximately a 90° angle to focus the energy around the tube ends **51.** More particularly, a "spot" of energy strikes the tube ends **51** and preferably, slightly exceed the diameter B of the tube **50** to ensure the tube is covered with adequate radiant energy.

Heat sensors **320** positioned in the housing **12** detect the temperature near the sealed ends **51.** Such heat sensors **320** may be, for example, thermopile infrared sensors. As the laser beam strikes the sealed tube ends **51,** the heating, melting and aseptic (and/or sterilization) process begins. Depending on the application, the sensors can be used to detect the desired temperature levels for melting and welding. For examples, some applications require aseptic conditions be generated. Typically, aseptic, high level disinfection, or germicidal conditions include a less than 6 log reduction of heat resistant spores. Other applications may require sterile conditions be generated. Sterile conditions generally include an operating mode of equal to or greater than a 6 log reduction of heat resistant spores.

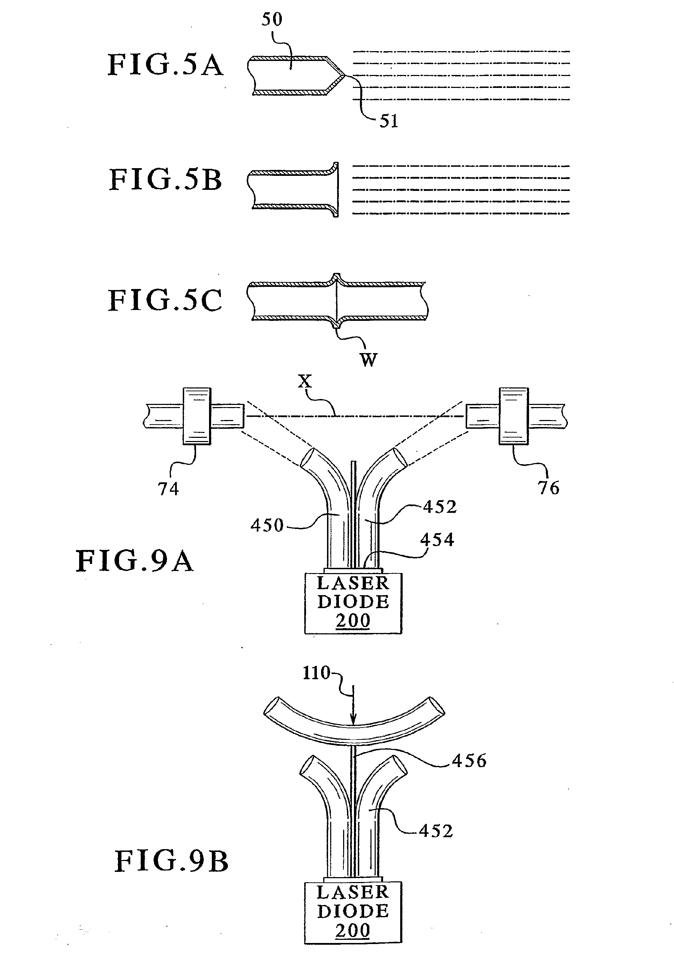

As the temperature of the tubing material at the tube ends **51** increases, the tube ends 51 begin to melt, flow and reopen. The tubing material has a certain level of "memory" - as the sealed end of the tube reopens, the tube is predisposed to returning to its symmetrical, circular form. FIG. 5A and 5B illustrate an example of the laser beam striking the sealed tube end **51.** As the laser beam strikes the tube end the rise in temperature at the tube end clauses the sealed end to peel open and flare. Once the heat sensors **320** detect that the required aseptic or sterilization temperature level is obtained and sufficient melting of the tube ends 51 has occurred the laser **200** shuts off

FIGS. 4D through 4F show the next step of the connecting process. After the laser 200 shuts off, the plate **106** moves to the back **16** of the housing **12.** As the plate **106** moves, the collimator **204** moves to the side 18 along track **107.** At the same time, the prism **206** moves toward the laser unit **200,** and the tube holders **70, 72** come together via track **105.** At this point, the now melted and aseptically heated or sterilized tube ends **51** contact each other. A weld-seal W is formed. Typically, the weld W is in the form of a ring as shown in FIG. 5C. The tube holders **70, 72** remain in this position until the weld W has sufficiently cooled. Alternatively, the laser unit **200** may be energized again (FIG. 4F). As shown in FIG. 4F, the laser beam is directed down the light pipe **220** to the tube ends. In this example, the weld-seal W forms and the laser unit **200** is shut off. The weld-seal W may be a hermetic seal.

In applications that use at least one "drum head" end, this type of end responds to the laser in a similar manner as that described above regarding the opening of a sealed end tube. One example of the "drum head" end is as follows. The film of the "drum had" may have a higher concentration of dye than the tubing material. This, the film heats faster than the tubing material. The film melts and flows outward to the perimeter of the tube and combines with the tube. The film material may be made from a variety of polymer materials such as polyolefins, polyamides, polyesters, styrene and hydrocarbon copolymers and particularly block copolymers of styrene and dienes and their hydrogenated derivatives, ethylene and vinyl acetate copolymers, ethylene and methacrylic acid copolymers and their ester derivatives. The film may be made from a blend of these materials and can be a monolayer or multiple layer structure. For example, polypropylene, polypropylene-Kraton blend, polypropylene polyethylene blend, or other compatible material.

Alternatives may include one tube holder that is stationary and one tube holder that moves within the apparatus.

### Weld inspection process

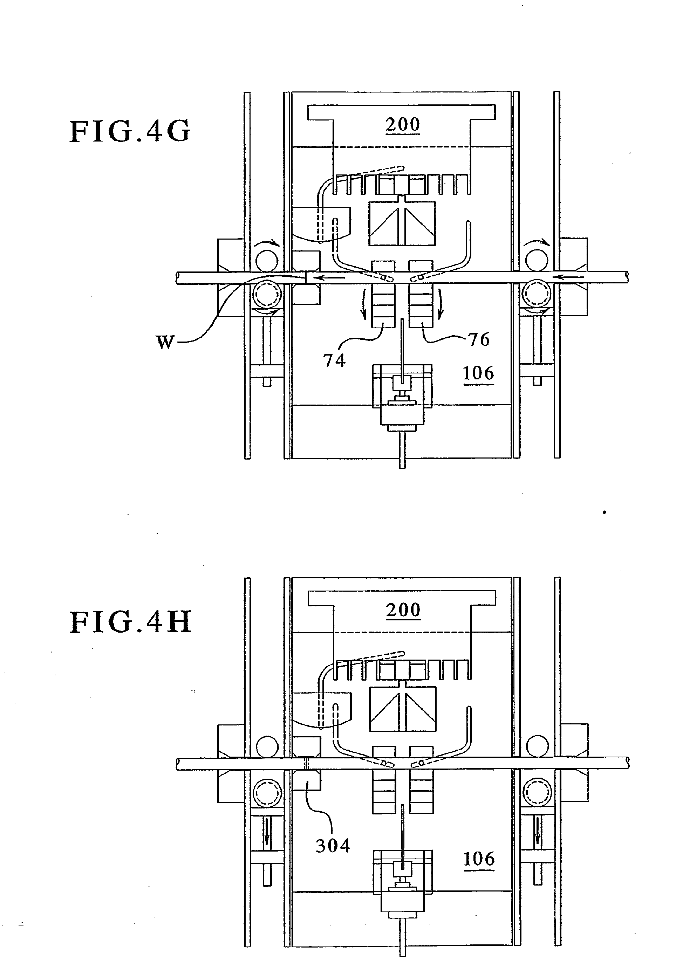

FIGS. 4G and 4H show the weld inspection process. Upon cooling, the first part **74, 78 of the** tube holders **70, 72** open and the guides **56, 58** move weld W to the weld detecting sensor 304 for the post process inspection- The inspection process analyzes, for example, the weld thickness and weld height. This data is compared to the profile data for an acceptable or "good" weld. This sensor **304** may be a CMOS image sensor. However, other similar image sensors may be employed. If the post process inspection indicates that the weld is a "good" weld, the lid **24** is unlocked and the guides **56, 58** opens. The user is free to open the lid **24** and remove the connected tubing.

On the other hand, if the post inspection process indicates a "bad" weld, the device automatically pinch seals the patient-side of the tubing. The automatic pinch seal process reduces the possibility of contaminants entering the tubing. During this situation, the user is notified of the "bad" weld. The user can then obtain another bag-side tube and start the connection process again. This unique inspection process provides a safety feature to ensure the patient uses "good" welds only. This is especially helpful for visually impaired patient who may have difficulty visually inspecting a weld after the connection process.

### Method of Disconnecting a Tube

FIGS. 2C and 2D generally illustrate the inventive method for disconnecting and sealing the tube **50.** When the user desires to disconnect from the dialysate solution bag, drainage bag, blood bag, or the like, he/she opens the lid **24** of the device **10.** When the lid **24** opens, the guides **56, 58** automatically move to the open position. (FIG. 2C, Ref. No. 56). The user places the tube **50** in the groove of the second part **78, 80** of the tube holders **70, 72.** In this way, the tube **50** extends along the funneled passageway **52, 54.** In this application, it is not necessary for the first part **74, 78** of the tube holders **70, 72** to close. The user closes the lid **24,** thus, closing the guides **56, 58** which, in turn, crimp the tubing **50.**

It is preferable to place the tube **50** so that the preexisting weld **W** is approximately centered between the tube holders **70, 72.** Similar to the connecting process, the sensor **300** identifies the tubing **50** and confirms that a patient-side tube is at one of the passageways **52, 54** and the bag-side tube is at the other passageway **52, 54.** Thus, the sensor **300** confirms that a preexisting weld exists somewhere there between, e.g., within the device.

After the sensors **300** accept the tubing, the same or different sensor **300** or **304** determines the location of the pre-existing weld **W** in the tube. This may be accomplished, for example, with a digital camera or similar device. The sensor searches for a flange in the weld **W.** Alternatively, the sensor **304** may detect a distinction in color between the tubing based on the color coding scheme such as that described above. Accordingly, the sensor **304** could identify a color change at the area surrounding the weld, indicating a weld **W** exists between the two different colors.

Once the pre-existing weld **W** is located, the sensor **304** identifies the position for the cut in the tube **50.** The guides **56, 58** are activated and the tube **50** moves a predetermined distance, on the catheter side, toward the patient. For example, the existing weld may be located at position **X.** The sensor **304** locates the weld and moves the tube X + 1/8" away from the patient side for the location of the crimping and separation of the tube. In this way, the position for the disconnection is a minimal distance from the existing weld. Therefore, waste of tube material of the patient catheter (or transfer set) is minimized. Alternatively, a patient extension line may be used between the transfer set and the disposable (or bag-side). The use of an extension line will prolong the life of the transfer set because the transfer set will not need to be replaced as often. Instead, the patient extension line is easily replaced by disconnecting the old extension line from the transfer set and connecting a new extension to the transfer set by the methods disclosed herein. Moreover, the sensor **304** ensures that the section of tube containing the existing weld is discarded. This improves the integrity of the remaining catheter tube. In addition, the sensor **304** provides a safety measure since making a weld on top of an existing weld may not be sufficiently durable.

At the start of the disconnecting process the laser unit **200** is off. As shown in FIG. 2D, the hammer **110** moves (via shaft **116**) into contact with the tube **50.** It should be noted that the hammer **110** is not heated prior to contacting the tube **50.** As the hammer **110** contacts the tube **50** it compresses the tube so that the inner surface of the tube is touching. To this extent, the hammer **110** pushes the liquid existing in the tube **50** out of the area to be disconnected.

The laser unit **200** is subsequently activated. The light pipe **220** directs the majority of the laser energy down it onto the tubing **50.** The tubing **50** continues to be pinched between the anvil **112** and the hammer **110.** In this example, the light pipe **220** is a part of the anvil **112.** As the heated tubing is pinched it begins to seal. The heat sensors **320** monitor the temperature near the pinched tubing. The laser unit **200** is shut off. In an embodiment, a sensor **320** is mounted on the hammer **110** near the front **111.** In general, the sensor verifies the laser is operating properly. The pinch hammer **110** remains in contact with the tubing while the tube cools. After the cooling of the tubes, the hammer **110** moves back to its original position. The guides **56, 58** are then activated and reverse movement a predetermined distance. This predetermined distance is dependent on the size and material of the flexible tube **50.** As the guides **56, 58** reverse, the tubing **50** is pulled apart resulting in two sealed ends. Thus, the combination of the guides **56, 58** and the hammer **110** act as a separator to separate the tubing into two sealed end tubes. The device **10** notifies the user that the lid **24** is unlocked and ready to be opened. The two newly sealed ends of tubing are subsequently unloaded from the device **10.** Other applications may include a laser that stays on for the duration or is pulsed on and off while the hammer moves in to pinch the tubing.

### Protective Film

FIG. 6 shows a protective film **400**. The protective film **400** covers the plano convex lenses **210, 212,** the anvil **112**, and the light pipe **220.** The protective film **400** is a thin clear material, preferably, a Mylar^{®} or polyethylene material. The film **400** is provided on, for example, a roll **402** that advances after each disconnection application. When the film **400** advances it is stored in another roll **404.** After the roll **402** is used both rolls **402** and **404** can be easily discarded. The laser energy does not have any heating effect on the film. The film **400** does not alter the laser beam characteristics. In this way, the film **400** protects the optics assembly **202** and eliminates cleaning of same. It will be appreciated that one could also achieve this purpose by providing a system of advancing disposable lenses. For example, if the optical assembly includes the light pipe **220** as the anvil **112,** the optical assembly could be a number of disposable lenses on a cartridge that rotates after each use. Thus, the used optical assembly is discarded and a new optical assembly is used in each application.

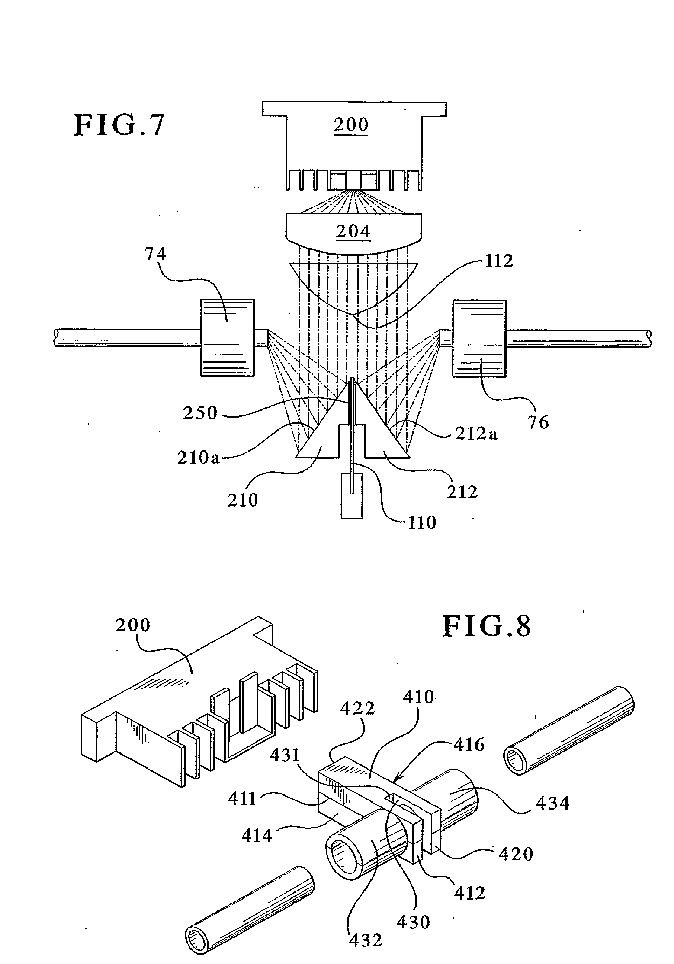

FIG. 7 illustrates another device in which the prism **206** and light pipe **220** are not between the tube holders **70, 72** but located near the front **14** of the housing **12.** For simplification purposes, FIG. 6 shows the anvil **112** between the collimator **204** and the prism **206.** However, during the connection process, the anvil **112** is generally not employed. Instead, the anvil **112** is off to one side **18** or **20** in the housing **12.** During the disconnection process, the anvil **112** moves in front of the laser **200**. Thus, the anvil **112** may be mounted on a tracking system similar to that described above with respect to the collimator **204.**

During the connection process, the prism lens 206 diffuses the laser beam and spreads the energy over a slightly large area than that described above in FIGS. 4A through 4H. In this example, the Jenses **210, 212** are shown with flat reflecting surfaces **210a, 212a.** However, it should be understood that the lenses **210, 212** may be concave or some other configuration depending on the laser type and the seed to redirect and focus the beam. Also, the prism **206** may be rough edged lenses **210, 212** to spread the energy at the surface of the sealed tube ends 51. Moreover, another lens (now shown) may be positioned at the surfaces **210a, 212a** between the surface and the tubing to further focus the laser beam. As described above with respect to FIGS. 2A and 2B, the tube ends are brought together after the tube ends are sufficiently heated and a weld is formed. However, the device of FIG. 6 is less complex because it is not necessary to move the prism 206 from in between the tube ends prior to bringing the tube ends together.

During the disconnecting process, the anvil **112** moves in front of the laser **200.** The hammer **110** moves through a passageway **250** between the two lenses **210, 212** in the direction of the anvil **112**. The hammer **110** compresses the tubing **50** against the anvil **112.** In this regard, the remaining steps of the disconnection process are substantially the same as that described above.

FIG. 8 shows an optical assembly. In this assembly, the laser optics assembly incorporates a fiber optics assembly **410.** The fiber optics assembly can include a large cylinder rod or multiple optical fibers to transmit the electromagnetic energy to the tube and provide the necessary heating and distribution of the energy. In this assembly, the fiber optics assembly **410** includes a fixed lens **412** with first and second sides **414, 416** and a front and back end **420, 422.** A recess or access slot **430** extends from the front end **420** into the assembly **410.** The recess **430** ends at wall **431** within the assembly **410.** The wall **431** acts as the anvil during the disconnection process. The fiber optics assembly **410** has a parting line **411** in which the assembly may be opened while the tube is loaded for the disconnection process. After the tube **50** is loaded, the recess **430** receives the hammer **110** and the hammer compresses the tube **50** at wall **431.** The laser unit **200** is energized and the laser beam is directed down the fixed lens **412** in a similar manner as the light pipe **220** described above. In this way, the crimping and separation process begins.

In addition, a fiber optic member **432, 434** extends perpendicular from each side **414, 416** of the lens **412.** During the connection process, the laser unit **200** is energized and the laser beam is directed down the fixed lens **412** to the fiber optic members **432, 434.** The fiber optic members **432, 434** emit the laser energy at the tube ends **50.** Similar to the embodiment described in FIGS. 2A and 2B above, the assembly **410** moves out from between the tube holders **70, 72** and the tube holders bring the tube ends together to form a weld. If necessary, the laser unit **200** can be energized again and the laser beam is directed down the fixed lens **412** to the area where the tube ends are joined to form a weld.

FIGS. 9A and 9B show another device. In FIG. 9A, an optical assembly **450** is used with the laser unit 200. The optical assembly **450** is adjacent to the laser unit **200** between the laser unit and a plane X that intersects the tubing **50.** The optical assembly **450** includes a generally "Y"-shaped optical splitter **452,** in which a base **454** of the "Y" is near the output of the laser unit **200.** The "Y"-shaped optical splitter extends from the laser unit **200** toward the plane X. The "Y"-shaped optics may be solid fiber optics or individual fibers.

The optical assembly **450** remains stationary during the connection and disconnection operation of the apparatus. During the connection of two tube ends **51,** the laser beam is split down the "Y" to each tube end. The tube ends are subsequently brought together for welding. During the disconnection process, the laser beam is directed down a center optical component of the assembly or light pipe **456.** Similar to the applications described above, the anvil **110** moves toward the light pipe **456** to compress and pinch the tubing. It is also within the scope of the invention to have the optics assembly **450** as two separate components. In this example, the "Y"-shaped optical splitter **452** and the light pipe **456** are discrete components (not shown). Each component **452, 456** is mounted on a movable plane that moves the required component in front of the laser unit depending on the process to be performed by the apparatus.

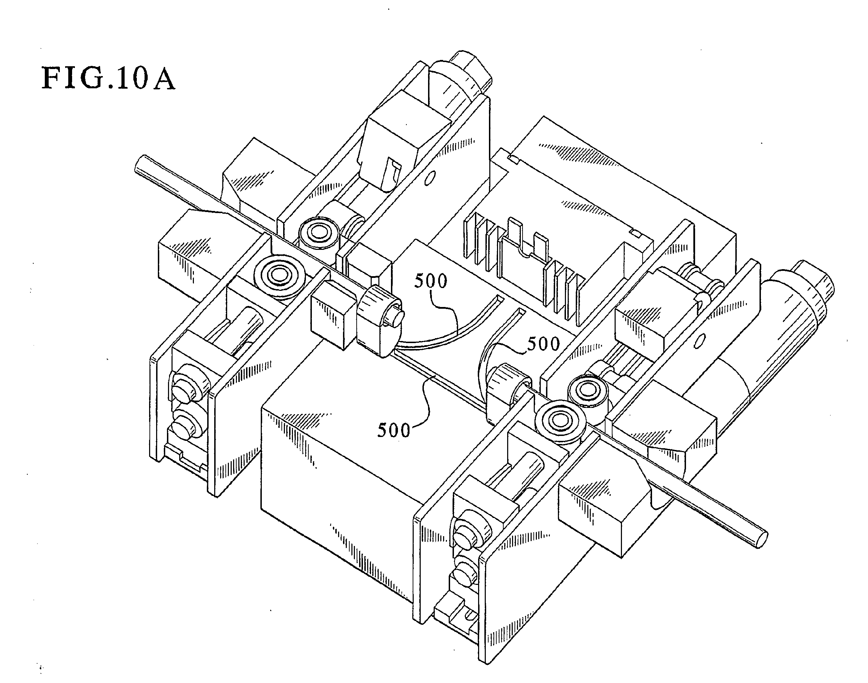

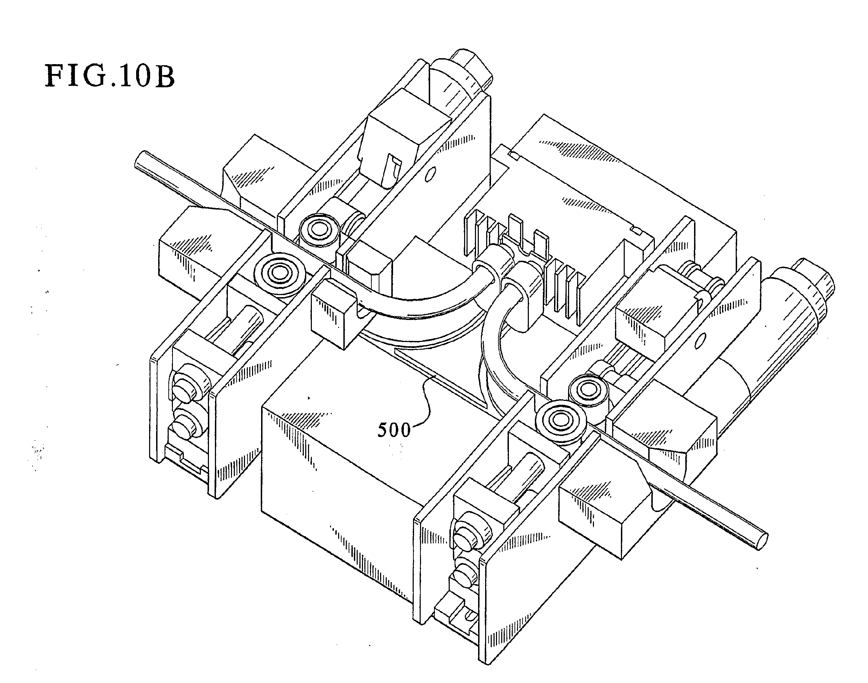

FIGS. 10A and 10B show another device. In this example, the laser unit **200** is used without the optics assembly. The tube holders **70, 72** are mounted on a track system **500.** During the connection process, the track system **500** moves the tube holders **70, 72** along a predetermined path toward the laser unit **200.** (FIG. 8B). In this way, the tube holders **70, 72** manipulate the tubes **50** so that the tube ends **51** are, preferably, parallel to each other and face the laser unit **200.** Thus, the tube holders **70, 72** rotate approximately **90** degrees from when they receive the tube ends to the point at which the tube ends **51** face the laser **unlit 200.** However, the tube holders may rotate in the range of **70** to **110** degrees and achieve the same results.

The laser unit **200** turns on and melts and sterilizes the tube ends. As discussed above, the tube ends **51** enter the device **10** as sealed tube ends. As such, the tube ends **51** begin to reopen as the laser energy melts this area. The laser unit **200** shuts off once the sensors determine that sufficient beating of the tube ends occurred. At this time, the tube holders **70, 72** retract to their starting position (near guides **56, 58**) and then move forward toward each other. The two tube ends **51** come into contact with each other and a weld seal is formed.

### Non-PVC, laser-weldable Tubing

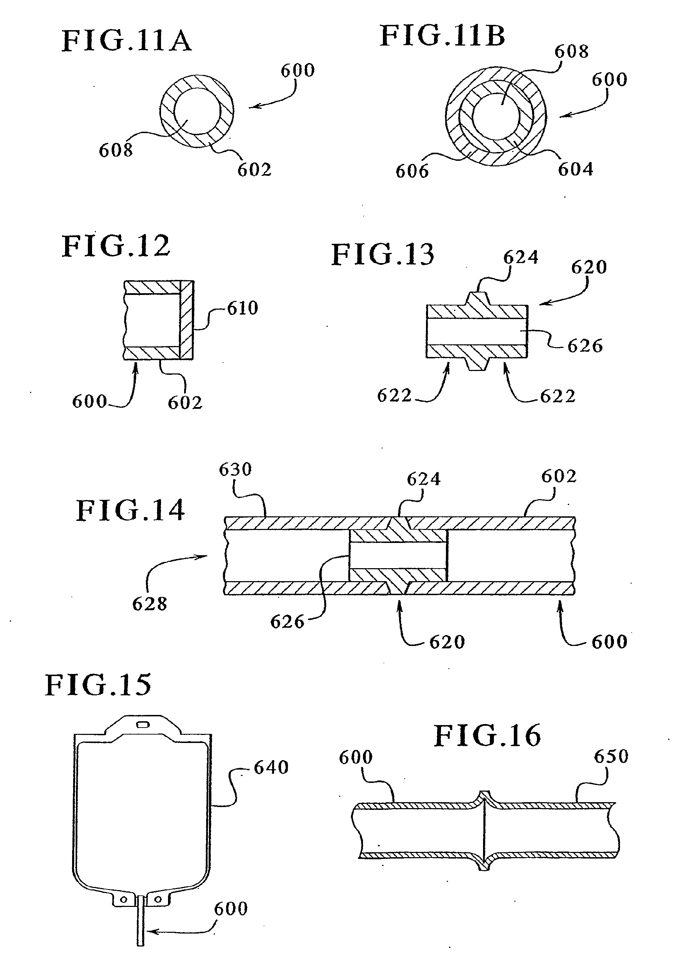

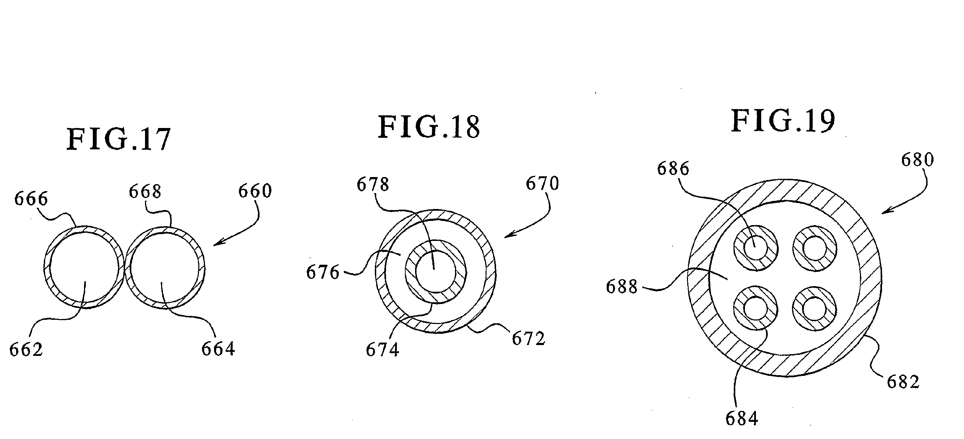

FIGS. 11a and 11b show a monolayer tubing **600** and a multiple layer tubing **600** respectively that are suitable for use with the present invention. FIG. 11c shows a multiple lumen tubing which can have two or more fluid passageways. Like the single lumen tubing of FIGS. 11a and 11b, the multiple lumen tubing of FIGS. 17-19 can be a monolayer structure or a multiple layer structure, and, therefore, it should be understood the following description shall apply to.single lumen tubings or multiple lumen tubings.

Also described herein the monolayer the tubing **600** has a sidewall **602** made from a polymeric material and more preferably from a non-PVC containing polymer and most preferably from a non-PVC containing polymer that is capable of heating upon exposure to a laser beam ("laser responsive"). The multiple layer tubing **600** has a first layer or solution contact layer **604** and a second layer **606.** At least one of the layers **604** or **606** is composed of a non-PVC containing polymer that is laser responsive. Also described herein, the other layer **604** or **606** will also be a non-PVC containing polymer, and more preferably a non-PVC containing polymer that heats upon exposure to a laser beam. However, it may also be desirable to have a solution contact layer **604** that is not laser responsive or does not contain any components that may leach into solution or react with the solution. Of course, it is contemplated that tubing having more than two-layers can be used. The tubing sidewalls define a fluid pathway **608** therethrough.

Suitable non-PVC containing polymers include polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers.

Suitable polyolefins include homopolymers and copolymers obtained by polymerizing alpha-olefins containing from 2 to 20 carbon atoms, and more preferably from 2 to 10 carbens. Therefore, suitable polyolefins include polymers and copolymers of propylene, ethylene, butene-1, pentene-1, 4-mefltyl-1-pentene, hexene-1, heptene-1, octene-1, nonene-1 and decene-1. Most preferably the polyolefin is a homopolymer or copolymer of propylene or a homopolymer or copolymer of polyethylene.

Suitable homopolymers of polypropylene can have a stereochemistry of amorphous, isotactic, syndiotactic, atactic, hemiisotactic or stereoblock. The polypropylene may have a low heat of fusion from about **20** joules/gram to about **220** joules/gram, more preferably from about **60** joules/gram to about **160** joules/gram and most preferably from about 80 joules/gram to about **130** joules/gram. It is also desirable, for the polypropylene homopolymer to have a melting point temperature of less than about 165°C and more preferably from about 130°C to about 160°C, more preferably from about 140°C to about 150°C. In one example the homopolymer of polypropylene is obtained using a single site catalyst.

Suitable copolymers of propylene are obtained by polymerizing a propylene monomer with an α-olefin having from 2 to 20 carbons. In one example the propylene is copolymerized with ethylene in an amount by weight from about 1% to about 20%, more preferably from about 1% to about 10% and most preferably from 2% to about 5% by weight of the copolymer. The propylene and ethylene copolymers may be random or block copolymers. The propylene copolymer should have a low heat of fusion of from about 40 joules/gram to about 140 joules/gram, more preferable from about 60 joules/gram to about 90 joules/gram. The propylene copolymer may be obtained using a single-site catalyst.

It is also possible to use a blend of polypropylene and α-olefin copolymers wherein the propylene copolymers can vary by the number of carbons in the α olefin. For example, blends of propylene and α-olefin copolymers are contemplated wherein one copolymer has a 2 carbon α-olefin and another copolymer has a 4 carbon α-olefin. It is also possible to use any combination of α-olefns from 2 to 20 carbons and more preferably from 2 to 8 carbons. In one example blends of propylene and α-olefin copolymers are contemplated wherein first and second α-olefins have the following combination of carbon numbers; 2 and 6, 2 and 8, 4 and 6, 4 and 8. It is also contemplated using more than 2 polypropylene and α-olefin copolymers in the blend. Suitable polymers can be obtained using a catalloy procedure.

It may also be desirable to use a high melt strength polypropylene. High melt strength polypropylenes can be a homopolymer or copolymer of polypropylene having a melt flow index within the range of 10 grams/10 min. to 800 grams/10 min., more preferably 30 grams/10 min. to 200 grams/10 min, or any range or combination of ranges therein. High melt strength polypropylenes are known to have free-end long chain branches of propylene units. Methods of preparing polypropylenes which exhibit a high melt strength characteristic have been described in U.S. Patent Nos. 4,916,198; 5,047,485; and 5,605,936. One such method includes irradiating a linear propylene polymer in an environment in which the active oxygen concentration is about 15% by volume with high energy ionization energy radiation at a dose of 1 to 10⁴ megarads per minute for a period of time sufficient for a substantial amount of chain scission of the linear propylene polymer to occur but insufficient to cause the material to become gelatinous. The irradiation results in chain scission. The subsequent recombination of chain fragments results in the formation of new chains, as well as joining chain fragments to chains to form branches. This further results in the desired free-end long chain branched, high molecular weight, non-linear, propylene polymer material. Radiation is maintained until a significant amount of long chain branches form. The material is then treated to deactivate substantially all the free radicals present in the irradiated material.

High melt strength polypropylenes can also be obtained as described in U.S. Patent No. 5,416,169, when a specified organic peroxide (di-2-ethylhexyl peroxydicarbonate) is reacted with a polypropylene under specified conditions, followed by melt-kneading. Such polypropylenes are linear, crystalline polypropylenes having a branching coefficient of substantially 1, and, therefore, has no free end long-chain branching and will have a intrinsic viscosity of from about 2.5 dl/g to 10 dl/g.

Suitable homopolymers of ethylene include those having a density of greater than 0.915 g/cc and includes low density polyethylene (LDPE), medium density polyethylene (MDPE) and high density polyethylene (HDPE).

Suitable copolymers of ethylene are obtained by polymerizing ethylene monomers with an α -olefin having from 3 to 20 carbons, more preferably 3-10 carbons and most preferably from 4 to 8 carbons. It is also desirable for the copolymers of ethylene to have a density as measured by ASTM D-792 of less than about 0.915 g/cc and more preferably less than about 0.910 g/cc and even more preferably less than about 0.900 g/cc. Such polymers are oftentimes referred to as VLDPE (very low density polyethylene) or ULDPE (ultra low density polyethylene). Preferably the ethylene α-olefin copolymers are produced using a single site catalyst and even more preferably a metallocene catalyst systems. Single site catalysts are believed to have a single, sterically and electronically equivalent catalyst position as opposed to the Ziegler-Natta type catalysts which are known to have a mixture of catalysts sites. Such single-site catalyzed ethylene α-olefins are sold by Dow under the trade name AFFINITY, DuPont Dow under the trademark ENGAGE® and by Exxon under the trade name EXACT. These copolymers shall sometimes be referred to herein as m-ULDPE.

Suitable copolymers of ethylene also include ethylene and lower alkyl acrylate copolymers, ethylene and lower alkyl substituted alkyl acrylate copolymers and ethylene vinyl acetate copolymers having a vinyl acetate content of from about 8% to about 40% by weight of the copolymer. The term "lower alkyl acrylates" refers to comonomers having the formula set forth in Diagram 1:

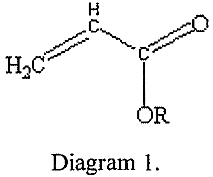

The R group refers to alkyls having from 1 to 17 carbons. Thus, the term "lower alkyl acrylates" includes but is not limited to methyl acrylate, ethyl acrylate, butyl acrylate and the like.

The term "alkyl substituted alkyl acrylates" refers to comonomers having the formula set forth in Diagram 2:

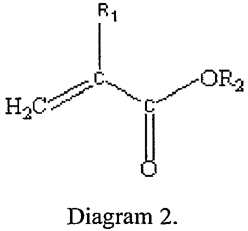

R₁ and R₂ are alkyls having 1-17 carbons and can have the same number of carbons or have a different number of carbons. Thus, the term "alkyl substituted alkyl acrylates" includes but is not limited to methyl methacrylate, ethyl methacrylate, methyl ethacrylate, ethyl ethacrylate, butyl methacrylate, butyl ethacrylate and the like.

Suitable polybutadienes include the 1,2- and 1,4-addition products of 1,3-butadiene (these shall collectively be referred to as polybutadienes). In a more preferred form of the invention the polymer is a 1,2-addition product of 1,3 butadiene (these shall be referred to as 1,2 polybutadienes). In an even more preferred form of the invention the polymer of interest is a syndiotactic 1,2-polybutadiene and even more preferably a low crystallinity, syndiotactic 1,2 polybutadiene. In a preferred form of the invention the low crystallinity, syndiotactic 1,2 polybutadiene will have a crystallinity less than 50%, more preferably less than about 45%, even more preferably less than about 40%, even more preferably the crystallinity will be from about 13% to about 40%, and most preferably from about 15% to about 30%. In a preferred form of the invention the low crystallinity, syndiotactic 1,2 polybutadiene will have a melting point temperature measured in accordance with ASTM D 3418 from about 70°C to about 120°C. Suitable resins include those sold by JSR (Japan Synthetic Rubber) under the grade designations: JSR RB 810, JSR RB 820, and JSR RB 830.

Suitable polyesters include polycondensation products of di-or polycarboxylic acids and di or poly hydroxy alcohols or alkylene oxides. In a preferred form of the invention, the polyester is a polyester ether. Suitable polyester ethers are obtained from reacting 1,4 cyclohexane dimethanol, 1,4 cyclohexane dicarboxylic acid and polytetramethylene glycol ether and shall be referred to generally as PCCE. Suitable PCCE's are sold by Eastman under the trade name ECDEL. Suitable polyesters further include polyester elastomers which are block copolymers of a hard crystalline segment of polybutylene terephthalate and a second segment of a soft (amorphous) polyether glycols. Such polyester elastomers are sold by Du Pont Chemical Company under the trade name HYTREL®.

Suitable polyamides include those that result from a ring-opening reaction of lactams having from 4-12 carbons. This group of polyamides therefore includes nylon 6, nylon 10 and nylon 12. Acceptable polyamides also include aliphatic polyamides resulting from the condensation reaction of di-amines having a carbon number within a range of 2-13, aliphatic polyamides resulting from a condensation reaction of di-acids having a carbon number within a range of 2-13, polyamides resulting from the condensation reaction of dimer fatty acids, and amide containing copolymers. Thus, suitable aliphatic polyamides include, for example, nylon 66, nylon 6,10 and dimer fatty acid polyamides.

The styrene of the styrene and hydrocarbon copolymer includes styrene and the various substituted styrenes including alkyl substituted styrene and halogen substituted styrene. The alkyl group can contain from 1 to about 6 carbon atoms. Specific examples of substituted styrenes include alpha-methylstyrene, beta-methylstyrene, vinyltoluene, 3-methylstyrene, 4-methylstyrene, 4-isopropylstyrene, 2,4-dimethylstyrene, o-chlorostyrene, p-chlorostyrene, o-bromostyrene, 2-chloro-4-methylstyrene, etc. Styrene is the most preferred.

The hydrocarbon portion of the styrene and hydrocarbon copolymer includes conjugated dienes. Conjugated dienes which may be utilized are those containing from 4 to about 10 carbon atoms and more generally, from 4 to 6 carbon atoms. Examples include 1,3-butadiene, 2-methyl-1,3-butadiene (isoprene), 2,3-dimethyl- 1,3-butadiene, chloroprene, 1,3-pentadiene, 1,3-hexadiene, etc. Mixtures of these conjugated dienes also may be used such as mixtures of butadiene and isoprene. The preferred conjugated dienes are isoprene and 1,3-butadiene.

The styrene and hydrocarbon copolymers can be block copolymers including di-block, tri-block, multi-block, and star block. Specific examples of diblock copolymers include styrene-butadiene, styrene-isoprene, and the hydrogenated derivatives thereof. Examples of triblock polymers include styrene-butadiene-styrene, styrene-isoprene-styrene, alpha-methylstyrene-butadiene-alpha-methylstyrene, and alpha-methylstyrene-isoprene-alpha-methylstyrene and hydrogenated derivatives thereof.

The selective hydrogenation of the above block copolymers may be carried out by a variety of well known processes including hydrogenation in the presence of such catalysts as Raney nickel, noble metals such as platinum, palladium, etc., and soluble transition metal catalysts. Suitable hydrogenation processes which can be used are those wherein the diene-containing polymer or copolymer is dissolved in an inert hydrocarbon diluent such as cyclohexane and hydrogenated by reaction with hydrogen in the presence of a soluble hydrogenation catalyst. Such procedures are described in U.S. Patent Nos. 3,113,986 and 4,226,952.

Particularly useful hydrogenated block copolymers are the hydrogenated block copolymers of styrene-isoprene-styrene, such as a styrene-(ethylene/propylene)-styrene block polymer. When a polystyrene-polybutadiene-polystyrene block copolymer is hydrogenated, the resulting product resembles a regular copolymer block of ethylene and 1-butene (EB). As noted above, when the conjugated diene employed is isoprene, the resulting hydrogenated product resembles a regular copolymer block of ethylene and propylene (EP). One example of a commercially available selectively hydrogenated is KRATON G-1652 which is a hydrogenated SBS triblock comprising 30% styrene end blocks and a midblock equivalent is a copolymer of ethylene and 1-butene (EB). This hydrogenated block copolymer is often referred to as SEBS. Other suitable SEBS or SIS copolymers are sold by Kurrarry under the tradename SEPTON® and HYBRAR®.

It may also be desirable to use graft modified styrene and hydrocarbon block copolymers by grafting an alpha, beta-unsaturated monocarboxylic or dicarboxylic acid reagent onto the selectively hydrogenated block copolymers described above.

The block copolymers of the conjugated diene and the vinyl aromatic compound are grafted with an alpha,beta-unsaturated monocarboxylic or dicarboxylic acid reagent. The carboxylic acid reagents include carboxylic acids per se and their functional derivatives such as anhydrides, imides, metal salts, esters, etc., which are capable of being grafted onto the selectively hydrogenated block copolymer. The grafted polymer will usually contain from about 0.1 to about 20%, and preferably from about 0.1 to about 10% by weight based on the total weight of the block copolymer and the carboxylic acid reagent of the grafted carboxylic acid. Specific examples of useful monobasic carboxylic acids include acrylic acid, methacrylic acid, cinnamic acid, crotonic acid, acrylic anhydride, sodium acrylate, calcium acrylate and magnesium acrylate, etc. Examples of dicarboxylic acids and useful derivatives thereof include malcic acid, maleic anhydride, fumaric acid, mesaconic acid, itaconic acid, citraconic acid, itaconic anhydride, citraconic anhydride, monomethyl maleate, monosodium maleate, etc.

The styrene and hydrocarbon block copolymer can be modified with an oil such as the oil modified SEBS sold by the Shell Chemical Company under the product designation KRATON G2705.

The tubing can be composed of a multiple component polymer blend. The polymer blend can include a polyolefin blended with a styrene and hydrocarbon copolymer. The polyolefin may be a propylene containing polymer and can be selected from the homopolymers and copolymers of propylene described above including high melt strength polypropylenes. It may also be desirable to have three or more components including a styrene and hydrocarbon copolymer with a blend of various types of polypropylenes. The polypropylene, either alone or in sum, can be present in an amount by weight of the blend from about 10% to about 50%, more preferably from about 15% to about 45% and most preferably from about 20% to about 40% with the balance of the blend being the styrene and hydrocarbon block copolymer.

When using oil modified SEBS it may be desirable, though not critical, to use a high melt strength polypropylene as a blend component. Suitable polypropylene and SEBS containing blends include: (1) precompounded blends of PP and SEBS sold by Wittenburg under the trade name CAWITON and particularly grades PR 3670E and PR4977; (2) from 90-98% by weight KRATON G2705 with 2-10% Basell PROFAX PF 611 high melt strength polypropylene; (3) 75% KRATON G2705 with 23% Basell PROFAX SA 861random copolymer of propylene and ethylene with 2% Basell PROFAX PF- 611 which is high melt strength PP; and (4) precompounded blend of PP/SEBS sold by J-Von under grade 70585 E.

In another preferred form of the invention, the tubing will be fabricated from a single m-ULDPE resin or a blend of m-ULDPE resins. One particularly suitable m-ULDPE resin is sold by DuPont-Dow under the trademark ENGAGE® and even more particularly ENGAGE® 8003 (density 0.885 g/cc). It is also contemplated blending more than one m-ULDPE resins. Such resins and tubings and film made therefrom are more fully set forth in U.S. Patent No. 6,372,848.

It is also contemplated fabricating tubing from polybutadienes or blends of polybutadiene resins described above.

Because the suitable non-PVC containing polymers and polymer blends are typically not laser responsive, one must incorporate into the polymer or polymer blend a laser responsive component. Suitable laser-responsive components include dyes, colorants and/or pigments. In a more preferred form of the invention, the laser responsive material is a dye and more preferably an organic dye having a functional group that is responsive to a laser beam at a wavelength, or a narrow range of wavelengths, within a range of wavelengths in the near infrared spectrum and more preferably from about 700 nm to about 1500 nm. Representative functional groups include polymethine, porphine, indanthrene, quinone, di- and tri-phenylmethane, and metal complexed dithiol dyes. In a preferred form of the invention, the dye will have an absorptivity of higher than about 50 (optical density/gram) when exposed to a laser beam providing light in the frequency range in which the dye is responsive. In a preferred form of the invention, the dye is responsive to a laser beam at peak wavelengths from about 780 nm to about 810 nm and generates sufficient heat over a short period of time to allow for melting of the non-PVC polymer or polymer blend. What is meant by short period of time is less than 15 seconds.

The dyes are preferably sparingly soluble or insoluble in an aqueous medium including water, saline solutions, dextrose solutions, lipid containing solutions and protein containing solutions so if they form a part of the solution contact layer they will not readily leach into the solution in a significant or deleterious amount. The dyes are also preferably thermally stable at temperatures reached during extrusion processing of the polymer or polymer blend. Suitable dyes are sold by Epolin Inc. under the trade name EPOLIGHT 4121 and 4149. When using a laser responsive material with an absorption of higher than about 50, only low quantities of such dye material is required and typically is added to the tubing blend in an amount from about 20 ppm to about 500 ppm, more preferably from about 100 ppm to about 400 ppm and most preferably from about 200 ppm to about 300 ppm. It is contemplated using a laser responsive material having an absorptivity of less than 50 but one would have to use higher concentrations of the laser responsive material. It is also contemplated using other dyes that are not responsive to the laser but are used for color coding purposes described above.

In another preferred form of the invention, the laser responsive material will be applied to a surface of materials to be joined instead of incorporating the laser responsive material into the blend. To this end, the laser responsive material is dissolved or suspended in a suitable carrier or solvent, and, in this form can be applied specifically to selected portions of the surfaces to be joined. The laser responsive material can be applied by dipping the surfaces to be joined into the laser responsive material, or the laser responsive material can be brushed on, sprayed on, printed on or the like.

The tubings of the present invention can be manufactured by any known polymer processing technique, but, in a preferred form of the invention, is formed by extrusion, coextrusion or injection molding. Such tubings are soft, flexible, kink resistant, have a good touch feeling (haptics), and are capable of being sterilized by steam sterilization, radiation or by ethylene oxide (EtO) exposure.

### Non-PVC, laser weldable end cap film

FIG. 12 shows the tubing 600 with an end cap 610 hermetically sealed thereto. The end cap film 610 is a monolayer or multiple layer polymeric film that has a tubing contacting surface 611 that is adhesively compatible with the tubing 600. The end cap film can be formed by any suitable polymer processing technique including extrusion, coextrusion, extrusion lamination, lamination, injection molding and the like. The end cap film 610, in a preferred form of the invention, is attached with sufficient strength to an end surface of tubing to withstand a burst strength of 206.8 KPa (30 psi). Burst strength is measured by applying pressurized air through a tubing flowpath 613 to pressurize the tubing until the tubing or end cap ruptures or leaks. The end cap 610 can be dimensioned to exceed the dimension of the end of the tubing and excess material is wrapped around the tubing end where it is attached to the tubing sidewalls 602 to form the drum embodiment referred to above. It is also possible to dimension the cap 610 as shown in FIG. 12 to match the dimension of the end of the tubing and be attached only to the end portion of the tubing without any significant amount of excess material.

The end cap 610 can be fabricated from single polymers such as the m-ULDPE resins described above. Such and end cap made from m-ULDPE are particularly well suited for use with m-ULDPE tubing containing blends. According to the present invention, the end cap **610** is fabricated from polymer blends having at least a first component and a second component. The first component is selected from: (1) ethylene and α-olefin copolymers having a density of less than about 0.915 g/cc, (2) ethylene and lower alkyl acrylate copolymers, (3) ethylene and lower alkyl substituted alkyl acrylate copolymers and (4) ionic polymers, commonly referred to as ionomers. The term "ionomer" is used herein to refer to metal salts of the acrylic acid copolymers having pendent carboxylate groups associated with monovalent or divalent cations such as zinc or sodium. The first component can be present in an amount from about 99% to about 50% by weight of the blend, more preferably from about 85%-50% and most preferably from about 70%-50%.

The second component is selected from the group consisting of (1) propylene containing polymers, (2) butene containing polymers, (3) polymethyl pentene containing polymers, (4) cyclic olefin containing polymers and (5) bridged polycyclic hydrocarbon containing polymers. The second component can be present in an amount by weight of the blend from about 50% to about 1%, more preferably from about 50%-15% and most preferably from about 30%-50%. These polymer blends for the end cap are particularly well suited for use with the tubings made from blends of polyolefin and styrene and hydrocarbon copolymers.

Suitable homopolymer and copolymers of cyclic olefins and bridged polycyclic hydrocarbons and blends thereof can be found in U.S. Patent Nos. 4,874,808; 5,003,019; 5,008,356; 5,288,560; 5,218,049; 5,854,349; 5,863,986; 5,795,945; and 5,792,824.

In a preferred form of the invention, suitable cyclic olefin monomers are monocyclic compounds having from 5 to about 10 carbons in the ring. The cyclic olefins can be selected from the group consisting of substituted and unsubstituted cyclopentene, cyclopentadiene, cyclohexene, cyclohexadiene, cycloheptene, cycloheptadiene, cyclooctene, and cyclooctadiene.

In a preferred form of the invention, suitable bridged polycyclic hydrocarbon monomers have two or more rings and more preferably contain at least 7 carbons. The rings can be substituted or unsubstituted. Suitable substitutes include lower alkyl, aryl, aralkyl, vinyl, allyloxy, (meth) acryloxy and the like. The bridged polycyclic hydrocarbons are selected from the group consisting of those disclosed in the above incorporated patents. Suitable bridged polycyclic hydrocarbon containing polymers are sold by Ticona under the tradename TOPAS, by Nippon Zeon under the tradename ZEONEX and ZEONOR, by Daikyo Gomu Seiko under the tradename CZ resin, and by Mitsui Petrochemical Company under the tradename APEL.

In a preferred form of the invention, the end cap will be a monolayer film from a blend by weight of from about 35% to about 45% of a ethylene and α-olefin copolymer having a density of less than about 0.900 g/cc, from about 20% to about 30% of an ethylene and α-olefin copolymer having a density of higher than about 0.900 g/cc but less than about 0.910 g/cc, and from about 30% to about 40% polypropylene, and more preferably a random copolymer of propylene with approximately 3% by weight of an ethylene comomoner. The end cap should have a thickness from about 0.08 mm to about 0.25 mm (about 3 mils to about 10 mils).

Because these end cap materials are not laser responsive, one of the laser responsive dyes must be incorporated into the single polymer or polymer blends for the end cap. For the end cap material, the dye should be incorporated in an amount from about 200 ppm to about 2000 ppm, more preferably from about 400 ppm to about 1800 ppm and most preferably from about 500 ppm to about 1000 ppm.

### Coupler

FIG. 13 shows a coupler 620 having opposed tubing mounting portions 622, a tubing stop 624 and a fluid pathway 626 therethrough. As shown in FIG. 14, the coupler can be used to connect a first tubing to a second tubing even when the first and second tubings are incompatible with one another. The assembly shown in FIG. 14 will be discussed in greater detail below. The tubing mounting portions 622 can have a tapered portion at its distal end for ease of mounting a tubing thereto. The surface of the coupler 620 can be textured or have a matte finish for ease of mounting of the tubing. The tubing mounting portions 622 are shown to have relatively the same length but could have different lengths without departing from the scope of the present invention. It is also contemplated the tubing mounting portions 622 can have ridges or other protuberances for heat concentrating or enhancing an interference fit between the tubing and the tubing mounting portions 622.

The tubing mounting portions 622 are shown to be concentrically mounted with respect to one another and with respect to the fluid pathway 626. It is contemplated the coupler can have numerous shapes where the tubing portions 622 are not concentrically disposed with respect to one another. It is contemplated one tubing mounting portion can have a first axis and the other tubing mounting portion will have a second axis transverse to the first axis. What is meant by transverse is one axis extends in a direction to intersect the second axis even if the axis will not intersect it. It is also contemplated the coupler can have more than two tubing mounting portions, more than one tubing stop and more than one fluid pathway. It is desirable the tubing when attached to the coupler have a bond strength in excess of 66.7 N (15 lbf) when tested by a pull test. An assembly fails the pull test if the tubing breaks or become detached from the coupler at a pull force below 66.7 N (15 lbf).

The coupler is composed of a non-PVC polymeric material selected from the materials set forth above for the tubing and preferably is composed of a polymer blend. In one preferred form of the invention the tubing has two components of from about 40% to about 60% EVA and a second component of from about 60% to about 40% of a polyester, polyester elastomer, or a polyurethane. The EVA, preferably, has a modifier group associated therewith and selected from the group consisting of: aromatic hydrocarbons, carbon dioxide, monoethylenically unsaturated hydrocarbons, acrylonitriles, vinyl ethers, vinyl esters, vinylamides, vinyl ketones, vinyl halides, epoxides, carboxylic acids and anhydride derivatives thereof (including fused ring carboxylic acid anhydrides). Most preferably, the modifier group is maleic acid or maleic anhydride.

In another preferred form of the invention the polymer blend has three components. The first component is a polyolefin and more preferably a propylene containing polymer in an amount by weight of from about 25% to about 35% such as those sold by Solvay under the trade name FORTILENE and most particularly FORTILENE grade KS 490. The second component is a polyester and more preferably a polyester elastomer such as those sold by DuPont under the tradename HYTREL® and in an amount by weight of the blend of from about 35% to about 45%. The second component can also be a polyurethane. The third component is an ethylene vinyl acetate copolymer having a vinyl acetate content of from about 8% VA to about 40% vinyl acetate, and more preferably a carboxylic acid modified EVA or a carboxylic acid anhydride modified EVA. The EVA is present in an amount by weight of the blend of from about 25% to about 35%. The present invention further contemplates acid modifying or carboxylic acid anhydride modifying the propylene first component instead of or in addition to such modification to the EVA. The coupler **620** can be made by polymer processing techniques such as injection molding.

Suitable polyurethanes include both aromatic and aliphatic type polyurethanes. Suitable polyurethanes are formed by reacting diisocyanate with a chain extender. Diisocyanates include: Diphenylmethane diisocyanate (MDI), toluene diisocyanate (TDI), hexylene diisocyanate (HDI), and isophorone diisocyanate (IPDI). Chain Extenders include diol type, diamine type, polyester containing polyols and polyether containing polyols. The diol type include: 1,4-butane diol, ethylene glycol, 1,6- hexane diol and 1,4-bis-beta-hydroxyethoxybenzene. The diamine type include aliphatic and aromatic type. Aliphatic type includes ethylene diamine. Aromatic type includes : toluylene diamine and diaminodiphenylmethane.

### Laser-weldable tubing assemblies and therapeutic fluid delivery sets

FIG. 14 shows a tubing assembly **628** having a first laser weldable tubing **600** and a second tubing **630** which are connected together and placed in fluid communication by the coupler **620**. The second tubing **630** is made from a material incompatible with the material of tubing **600**. What is meant by incompatible is the materials are not capable of being directly joined together in a secure fashion utilizing conductive or inductive heat sealing techniques. This assembly **628** is particularly well suited for joining a PVC tubing **630** from a fluid delivery set or peritoneal dialysis set with the laser weldable, non-PVC tubing **600** described above. The laser weldable tubing **600** can be connected to a transfer set of a patient using the laser welding device **10** discussed above.

The first and second tubing **600, 630** can be connected to the coupler by sliding the fluid pathway **608** of the tubings over the tubing mounting portions **622** until an end portion of the tubing contacts the tubing stop **624.** The tubings **600** and **630** are then fixedly attached to the coupler by heat sealing such as with a ring-shaped die, radio frequency heat sealing, solvent bonding, adhesive bonding, or by heating the tubing and coupler in an autoclave during a steam sterilization process (i.e., 121°C for 1 hour) or other suitable techniques. When heat sealing a mandrel may be employed to maintain the shape of the coupler and tubing assembly.

FIG. 15 shows a fluid container **640** such as a therapeutic fluid container for storing a dialysate solution, a drain bag of a peritoneal dialysis set, an I.V. container, a blood container, a blood component container a blood substitute container or the like, in fluid communication with a laser weldable tubing **600**.

FIG. 16 shows the laser-weldable tubing **600** connected to a tubing **650** from a peritoneal dialysis transfer set. The tubing connection is made with the device **10** as described in detail above.

FIG. 17 shows a laser-weldable dual lumen tubing **660** having first and second fluid passageways **662** and **664** and first and second lumen **666** and **668** attached together. It is contemplated that more than two lumen, such as three, four or five or more, could be attached together without departing from the scope of the present invention. The first and second lumen are connected along peripheral edges and can extend parallel with respect to one another along a length of the tubing or the first and second lumen can be helically disposed with respect to one another or otherwise braided.

FIG. 18 shows another embodiment of a laser-weldable dual lumen tubing **670** having a first and second lumen **672** and **674** having, respectively, first and second fluid passageways **676** and **678**. The first and second lumen **672** and **674** are concentrically disposed with respect to one another. It is contemplated that more than two lumen could be concentrically mounted without departing from the present invention.

FIG. 19 shows yet another embodiment of a laser-weldable, multiple lumen tubing **680** having a primary lumen **682** and four secondary lumen **684** each having a fluid passageway **686**. The term "multiple" is meant to include two or more so a dual lumen tubing is a multiple lumen tubing. The area **688** between the secondary lumen **684** can be a fluid passageway or can be material such as packing material to hold the secondary lumen in position. While four secondary lumen are shown it is contemplated that two or more secondary lumen could be provided without departing from the present invention. Also, while the secondary lumen **684** are shown spaced apart from one another one or more of these secondary lumen can be attached together. The secondary lumen **684** can extend in a direction parallel to one another or be helically disposed with respect to one another or otherwise braided together.

### Examples

Example 1: A membrane film was extruded from a polymer blend of 40% by weight of a first m-ULDPE resin (density 0.885 g/cc, Dow VP 8770), 25% by weight of a second m-ULDPE resin (density of 0.902 g/cc, Dow PL 1880), 35% polypropylene (Basell SR-549M) and 600 ppm dye (Epolin 4121). The components were blended in a David Standard twin screw extruder and extruded through a die to a thickness of 0.13 mm (0.005 inches). The film was exposed to a diode laser (δ10 nm), 30 amp for a period of 10 seconds where the film melted.

Example 2: A tubing was extruded from a polymer blend of Cawiton PR 3670 with 200 ppm dye (Epolin 4121). The components were blended in a David Standard twin screw extruder and extruded through a die to a wall thickness 0.1 mm (0.039 inches) ID of 4 mm (0.157 inches), OD of 6 mm (0.235 inches). The tubing was exposed to a diode laser (810 nm), 30 amp for a period of 10 seconds where the tubing melted.

Example 3: A tubing was extruded in the same manner as in Example 2 except the blend contained 250 ppm of the dye. The tubing was exposed to a diode laser (810 nm), 30 amp for a period of 10 seconds where the tubing melted.

Example 4: A coupler was injection molded from a two component polymer blend of 50% HYTREL® 5556 WITH 50% BYNEL 1123. The blend components were pellitized with a 38 mm (1½ inch). David Standard twin screw extruder and injected molded with a 25 ton Arburg injection molding machine. A first tubing of PVC was slid over a first tubing mounting portion and attached to the coupler by radio frequency sealing. A second tubing was fabricated as set forth in Example 2. The second tubing was slid over a second tubing mounting portion of the coupler and then autoclaved at 121°C for one hour. The assembly was allowed to cool and the first tubing and the second tubing were pulled until the tubing broke or until it became detached from the coupler and the force required to do so was measured respectively at 130.3 N and 130.7 N (29.3 lbf and 29.4 lbf).

Example 5: A coupler was injection molded as set forth in Example 4 from a three-component polymer blend of 30% polypropylene (Solvay KS 490), 40% polyester elastomer (HYTREL® 5556) and 30% anhydride modified EVA (BYNEL 3810). As in example 4, a first tubing of PVC was slid over a first tubing mounting portion and sealed thereto using radio frequency sealing. A second tubing was fabricated as set forth in Example 2 and was slid over a second tubing mounting portion. The assembly was autoclaved at 121°C for one hour. The assembly was allowed to cool and the first tubing and the second tubing were pulled until the tubing broke or until it became detached from the coupler and the force required to do so was measured respectively at 214.4 N and 152.6 N (48.2 lbf and 34.3 lbf).

It should be understood that various changes and modifications to the presently preferred embodiments described herein will be apparent to those skilled in the art. Such changes and modifications can be made without departing from the scope of the present invention and without diminishing its intended advantages. It is therefore intended that such changes and modifications be covered by the appended claims.

## Claims

1. A tubing assembly comprising:
(a) a tubing having a fluid outlet and a sidewall comprising a layer of a polymer blend comprising a first component which is a material that is not thermally responsive to laser beam and is selected from polyolefins, ethylene and lower alkyl acrylate copolymers, ethylene and lower alkyl substituted alkyl acrylate copolymers, ethylene vinyl acetate copolymers, polybutadienes, polyesters, polyamides, and styrene and hydrocarbon copolymers; a second component of a laser responsive material having low solubility in aqueous medium; and the blend melts upon exposure to a laser beam having a wavelength of from 700 nm to 1500 nm for a short period of time; and
(b) an and cap film covering the fluid outlet which end cap film is hermetically sealed to the tubing and which film is a polymeric material having a monolayer structure or a multilayer structure, wherein the polymeric material is a blend comprising: a first component selected from (1) ethylene and α-olefin copolymers having a density of less than about 0.915 g/cc. (2) ethylene and lower alkyl acrylate copolymers, (3) ethylene and lower alkyl substituted alkyl acrylate copolymers and (4) ionic polymers; and a second component selected from the group consisting of: (1) propylene containing polymers, (2) butene containing polymers, (3) polymethyl pentene containing polymers, (4) cyclic olefin containing polymers and (5) bridged polycyclic hydrocarbon containing polymers.

2. An assembly according to claim 1, wherein the polymeric material further comprises a laser responsive material.

3. An assembly according to claim 2, wherein the laser responsive material has a functional group selected from the group polymethine, porphine, indanthrene, quinone, di-and tri-phenylmethane, and metal complexed dithiol dyes.

4. A medical fluids delivery assembly comprising:
a container for storing a therapeutic fluid; and
a tubing as defined in part (a) of claim 1 connected to the container.

5. An assembly according to claim 4, comprising a therapeutic fluid selected from peritoneal dialysis solutions, spent peritoneal dialysis solutions, nutritional solutions, blood, blood components, blood substitutes and IV solutions.

6. An assembly according to claim 4, further comprising an end cap film forming a fluid tight seal on an end portion of the tubing.

7. An assembly according to claim 4, wherein the first component of the polymer blend is a polyolefin obtained from a monomer of an α-olefin having from 2 to 20 carbons.

8. An assembly according to claim 7, wherein the polyolefin is selected from ethylene containing polymers, homopolymers of polypropylene and copolymers of polypropylene.

9. An assembly according to claim 8, wherein the copolymer of polypropylene is a random copolymer or a block copolymer.

10. An assembly according to claim 9, wherein the copolymer of polypropylene is obtained by polymerizing a propylene monomer with an α-olefin having from 2 to 20 carbons, or is a random copolymer with ethylene or a block copolymer with ethylene.

11. An assembly according to claim 4, wherein the first component of the polymer blend is a blend of a polypropylene and a styrene and hydrocarbon copolymer.

12. An assembly according to claim 11, wherein the styrene and hydrocarbon copolymer is a random copolymer of styrene and hydrocarbon or block copolymer of styrene and hydrocarbon.

13. An assembly according to claim 12, wherein the styrene and hydrocarbon block copolymer Is a di-block copolymer, tri-block copolymer, multi-block copolymer, or a star block copolymer.

14. A tubing assembly comprising:
(a) a first tubing of a material containing polyvinyl chloride;
(b) a second tubing as defined in part (a) of claim 1 which does not bond well directly to the first tubing; and
(c) a coupler joining the first tubing to the second tubing in fluid communication,

15. A medical fluid delivery tubing set assembly comprising;
a tubing assembly as defined in claim 14 and a third tubing which is connected to the second tubing and is adapted to be placed in fluid communication with a patient; wherein the first tubing is in fluid communication with a source of a therapeutic fluid.

16. An assembly according to claim 1, wherein the second component of the polymer blend of the tubing is present in an amount of from 20 ppm to 500 ppm.

17. An assembly accorcding to claim 1, wherein the end cap film comprises:
a layer comprising a first blend of a polymeric component and a laser responsive component, wherein the polymeric component is a second blend of from 99% to 50% by weight of the first component and from 50% to 1 % of the second component: and
wherein the film melts upon exposure to a laser beam having a wave length from 700nm to 1500 nm for a short period of time.

18. An assembly according to claim 1 , wherein the polymeric component comprises by weight of from 35% to 45% of a first ethylene and α-olefin copolymer having a density of less than about 0.900 g/cc, from 20% to 30% of a second ethylene and α-olefin copolymer having a density of higher than about 0.900 g/cc but less than about 0.910 g/cc, and from 30% to 40% of a polypropylene.

## Patentansprüche

1. Schlauchanordnung, umfassend:
a) Schlauch mit einem Flüssigkeitsausgang und einer Seitenwand, umfassend eine Schicht eines Polymergemisches, umfassend einen ersten Bestandteil, das ein Material ist, welches nicht thermisch reaktionsfähig gegenüber einem Laserstrahl ist, und aus Polyolefinen, Ethylen- und Niederalkylacrylat-Copolymeren, Ethylen- und Niederalkyl-substituierten Alkylacrylat-Copolymeren, Ethylenvinylacetat-Copolymeren, Polybutadienen, Polyestern, Polyamiden und Styrol- und Kohlenwasserstoff-Copolymeren ausgewählt ist; einen zweiten Bestandteil eines laserreaktiven Materials mit geringer Löslichkeit im wässrigen Medium; und das Gemisch schmilzt bei Einwirkung eines Laserstrahls mit einer Wellenlänge von 700 nm bis 1500 nm für eine kurze Zeit; und
b) eine Verschlusskappenfolie, den Flüssigkeitsausgang bedeckend, die Verschlusskappenfolie ist hermetisch mit dem Schlauch versiegelt und die Folie ist ein Polymermaterial mit einer Einzelschichtstruktur oder einer Mehrschichtstruktur, wobei das Polymermaterial ein Gemisch ist, umfassend: einen ersten Bestandteil, ausgewählt aus (1) Ethylen- und α-Olefin-Copolymeren mit einer Dichte von weniger als etwa 0,915 g/cc, (2) Ethylen- und Niederalkylacrylat-Copolymeren, (3) Ethylen- und Niederalkyl-substituierten Alkylacrylat-Copolymeren und (4) ionischen Polymeren; und einen zweiten Bestandteil, ausgewählt aus der Gruppe, bestehend aus: (1) Propylen-haltigen Polymeren, (2) Buten-haltigen Polymeren, (3) Polymethylpenten-haltigen Polymeren, (4) cyclisches Olefin-haltigen Polymeren und (5) verbrückte polycyclische Kohlenwasserstoff-haltigen Polymeren.

2. Anordnung nach Anspruch 1, wobei das Polymermaterial weiter ein laserreaktives Material umfasst.

3. Anordnung nach Anspruch 2, wobei das laserreaktive Material eine funktionelle Gruppe, ausgewählt aus der Gruppe Polymethin, Porphin, Indanthren, Chinon, Di- und Triphenylmethan und metallkomplexierten Dithiolfarbstoffen, aufweist.

4. Medizinische Flüssigkeitsbeförderungsanordnung, umfassend:
einen Behälter für die Aufbewahrung einer therapeutischen Flüssigkeit; und
einen Schlauch wie definiert in Teil a) des Anspruchs 1, verbunden mit dem Behälter.

5. Anordnung nach Anspruch 4, umfassend eine therapeutische Flüssigkeit, ausgewählt aus Peritonealdialyselösungen, verbrauchten Peritonealdialyselösungen, Ernährungslösungen, Blut, Blutbestandteilen, Blutersatzmitteln und IV-Lösungen.

6. Anordnung nach Anspruch 4, weiter umfassend eine Verschlusskappenfolie, eine flüssigkeitsdichte Versiegelung an einem Endstück des Schlauchs bildend.

7. Anordnung nach Anspruch 4, wobei der erste Bestandteil des Polymergemisches ein Polyolefin ist, erhalten aus einem Monomer eines α-Olefins mit 2 bis 20 Kohlenstoffatomen.

8. Anordnung nach Anspruch 7, wobei das Polyolefin aus Ethylen-haltigen Polymeren, Homopolymeren von Polypropylen und Copolymeren von Polypropylen ausgewählt ist.

9. Anordnung nach Anspruch 8, wobei das Copolymer von Polypropylen ein statistisches Copolymer oder ein Blockcopolymer ist.

10. Anordnung nach Anspruch 9, wobei das Copolymer von Polypropylen durch Polymerisation eines Propylenmonomers mit einem α-Olefin mit 2 bis 20 Kohlenstoffatomen erhalten wird oder ein statistisches Copolymer mit Ethylen oder ein Blockcopolymer mit Ethylen ist.

11. Anordnung nach Anspruch 4, wobei der erste Bestandteil des Polymergemisches ein Gemisch eines Polypropylens und eines Styrol- und Kohlenwasserstoff-Copolymers ist.

12. Anordnung nach Anspruch 11, wobei das Styrol- und Kohlenwasserstoff-Copolymer ein statistisches Copolymer von Styrol und Kohlenwasserstoff oder ein Blockcopolymer von Styrol und Kohlenwasserstoff ist.

13. Anordnung nach Anspruch 12, wobei das Styrol- und Kohlenwasserstoff-Blockcopolymer ein Diblockcopolymer, ein Triblockcopolymer, ein Multiblockcopolymer oder ein sternförmiges Blockcopolymer ist.

14. Schlauchanordnung, umfassend:
(a) einen ersten Schlauch eines Materials, Polyvinylchlorid enthaltend;
(b) einen zweiten Schlauch wie definiert in Teil (a) des Anspruchs 1, der nicht gut direkt mit dem ersten Schlauch bindet; und
(c) ein Kupplungsstück, den ersten Schlauch mit dem zweiten Schlauch zu einer Flüssigkeitsübertragung verbindend.

15. Medizinische Flüssigkeitsbeförderungschlauchsatzsanordnung, umfassend:
eine Schlauchanordnung wie definiert in Anspruch 14 und einen dritten Schlauch, der mit dem zweiten Schlauch verbunden ist, und angepasst ist, in Flüssigkeitsübertragung mit einem Patienten zu treten; wobei der erste Schlauch in Flüssigkeitsübertragung mit einer Quelle einer therapeutischen Lösung ist.

16. Anordnung nach Anspruch 1, wobei der zweite Bestandteil des Polymergemisches des Schlauches mit einem Anteil von 20 ppm bis 500 ppm vorliegt.

17. Anordnung gemäß Anspruch 1, wobei die Verschlusskappenfolie umfasst:
eine Schicht, umfassend ein erstes Gemisch eines Polymerbestandteils und eines laserreaktiven Bestandteils, wobei der Polymerbestandteil ein zweites Gemisch von 99 Gew.-% bis 50 Gew.-% des ersten Bestandteils und von 50% bis 1% des zweiten Bestandteils ist; und
wobei die Folie bei Einwirkung eines Laserstrahls mit einer Wellenlänge von 700 nm bis 1500 nm für eine kurze Zeit schmilzt.

18. Anordnung gemäß Anspruch 1, wobei der Polymerbestandteil 35 Gew.-% bis 45 Gew.-% eines ersten Ethylen- und α-Olefin-Copolymers mit einer Dichte von weniger als etwa 0,900 g/cc, 20 Gew.-% bis 30 Gew.-% eines zweiten Ethylen- und α-Olefin-Copolymers mit einer Dichte höher als etwa 0,900 g/cc, aber weniger als etwa 0,910 g/cc und 30 Gew.-% bis 40 Gew.-% eines Polypropylens umfasst.

## Revendications

1. Ensemble de tuyauterie comprenant :
(a) une tuyauterie comportant une sortie de fluide et une paroi latérale comprenant une couche d'un mélange de polymères comprenant un premier composant qui est un matériau qui n'est pas thermiquement sensible à un faisceau laser et est sélectionné parmi des polyoléfines, des copolymères d'éthylène et acrylate d'alkyle inférieur, des copolymères d'éthylène et acrylate d'alkyle substitué par alkyle inférieur, des copolymères d'éthylène et acétate de vinyle, des polybutadiènes, des polyesters, des polyamides, et des copolymères de styrène et hydrocarbure ; un second composant en un matériau sensible au laser présentant une faible solubilité en milieu aqueux ; et le mélange fond lors de l'exposition à un faisceau laser présentant une longueur d'onde de 700 nm à 1500 nm pendant une courte durée ; et
b) un film d'obturation d'extrémité recouvrant la sortie de fluide, lequel film d'obturation d'extrémité est scellé hermétiquement sur la tuyauterie et lequel film est en un matériau polymère présentant une structure monocouche ou une structure multicouche, dans lequel le matériau polymère est un mélange comprenant : un premier composant sélectionné parmi (1) des copolymères d'éthylène et α-oléfine présentant une densité inférieure à 0,915 g/cm3 environ, (2) des copolymères d'éthylène et acrylate d'alkyle inférieur, (3) des copolymères d'éthylène et acrylate d'alkyle substitué par alkyle inférieur et (4) des polymères ioniques ; et un second composant sélectionné parmi un groupe constitué par : (1) des polymères contenant du propylène, (2) des polymères contenant du butène, (3) des polymères contenant du polyméthyle-pentène, (4) des polymères contenant une oléfine cyclique et (5) des polymères contenant des hydrocarbures polycycliques pontés.

2. Ensemble selon la revendication 1, dans lequel le matériau polymère comprend en outre un matériau sensible au laser.

3. Ensemble selon la revendication 2, dans lequel le matériau sensible au laser comporte un groupe fonctionnel sélectionné parmi le groupe des colorants à base de polyméthine, porphine, indanthrène, quinone, di- et tri-phénylméthane et de di-thiol à complexe métallique.

4. Ensemble d'administration de fluides médicaux comprenant :
un conteneur destiné à stocker un fluide thérapeutique ; et
une tuyauterie telle que définie en partie (a) de la revendication 1, raccordée au conteneur.

5. Ensemble selon la revendication 4, comprenant un fluide thérapeutique sélectionné parmi des solutions de dialyse péritonéale, des solutions de dialyse péritonéale usagées, des solutions nutritionnelles, du sang, des composants sanguins, des substituts sanguins et des solutions de perfusion (IV).

6. Ensemble selon la revendication 4, comprenant en outre un film d'obturation d'extrémité formant un joint étanche au fluide sur une partie d'extrémité de la tuyauterie.

7. Ensemble selon la revendication 4, dans lequel le premier composant du mélange de polymères est une polyoléfine obtenue à partir d'un monomère et d'une α-oléfine comportant de 2 à 20 atomes de carbone.

8. Ensemble selon la revendication 7, dans lequel la polyoléfine est sélectionnée parmi des polymères contenant de l'éthylène, des homopolymères de polypropylène et des copolymères de polypropylène.

9. Ensemble selon la revendication 8, dans lequel le copolymère de polypropylène est un copolymère aléatoire ou un copolymère à blocs.

10. Ensemble selon la revendication 9, dans lequel le copolymère de polypropylène est obtenu par polymérisation d'un monomère de propylène avec une α-oléfine comportant de 2 à 20 atomes de carbone, ou est un copolymère aléatoire avec de l'éthylène ou un copolymère à blocs avec de l'éthylène.

11. Ensemble selon la revendication 4, dans lequel le premier composant du mélange de polymères est un mélange d'un polypropylène et d'un copolymère de styrène et hydrocarbure.

12. Ensemble selon la revendication 11, dans lequel le copolymère de styrène et hydrocarbure est un copolymère aléatoire de styrène et hydrocarbure ou un copolymère à blocs de styrène et hydrocarbure.

13. Ensemble selon la revendication 12, dans lequel le copolymère à blocs de styrène et hydrocarbure est un copolymère bi-bloc, un copolymère tri-bloc, un copolymère multi-bloc, ou un copolymère à blocs en étoile.

14. Ensemble de tuyauterie comprenant ;
(a) une première tuyauterie en un matériau contenant du chlorure de polyvinyle ;
(b) une deuxième tuyauterie telle que définie dans la partie (a) de la revendication 1 qui ne se soude pas directement à la première tuyauterie ; et
(c) un raccord reliant la première tuyauterie à la deuxième tuyauterie en communication fluidique.

15. Ensemble d'administration de fluide médical comprenant :
un ensemble de tuyauterie selon la revendication 14 et une troisième tuyauterie qui est raccordée à la deuxième tuyauterie et est adaptée de manière à être placée en communication fluidique avec un patient ; dans lequel la première tuyauterie est en communication fluidique avec une source d'un fluide thérapeutique.

16. Ensemble selon la revendication 1, dans lequel le second composant du mélange de polymères de la tuyauterie est présent en une quantité de 20 ppm à 500 ppm.

17. Ensemble selon la revendication 1, dans lequel le film d'obturation d'extrémité comprend :
une couche comprenant un premier mélange d'un composant polymère et d'un composant sensible au laser, dans lequel le composant polymère est un second mélange de 99 % à 50 % en poids du premier composant et de 50 % à 1 % du second composant ; et
dans lequel le film fond lors de l'exposition à un faisceau laser présentant une longueur d'onde de 700 nm à 1500 nm pendant une faible durée.

18. Ensemble selon la revendication 1, dans lequel le composant polymère comprend, en poids, de 35 % à 45 % d'un premier copolymère d'éthylène et α-oléfine présentant une densité inférieure à 0,900 g/cm3 environ, de 20 % à 30 % d'un second copolymère d'éthylène et α-oléfine présentant une densité supérieure à 0,900 g/cm3 environ mais inférieure à 0,910 g/cm3 environ, et de 30 % à 40 % d'un polypropylène.
